# EUROPEAN PATENT APPLICATION

(11) **EP 3 738 614 A1**
(43) Date of publication of application: **18.11.2020**
(21) Application number: 19382371.3
(22) Date of filing: 13.05.2019
(51) Int. Cl.: A61K 47/68

(54) **METHODS FOR PRODUCING HOMOGENEOUS ANTIBODY DRUG CONJUGATES**

(71) Applicant: Farmhispania S.A., 08160 Montmelo (ES)
(72) Inventor: CAMPS REÑÉ, Marc, 08160 Montmeló (ES); FARRÀS COSTA, Mercè, 08160 Montmeló (ES); MARTÍNEZ PÉREZ, Óscar, 08160 Montmeló (ES); PUJOL OLLÉ, Xavier, 08160 Montmeló (ES)
(74) Representative: ZBM Patents ApS

(57) **Abstract**

A method is provided for producing antibody conjugates having homogeneous payload antibody ratio. The method comprises: (i) providing separate fractions of heavy chains and light chains of an antibody or a fragment thereof both having interchain thiols; (ii) separately conjugating a payload to the interchain thiols of the heavy chains or to the interchain thiols of the light chains or to both; and (iii) assembling the resulting heavy chains with the resulting light chains by mixing them to form antibody conjugates. A homogeneous composition is obtained comprising antibody conjugates of the same payload antibody ratio, wherein the antibody in the antibody conjugate has its native amino acid sequence without modifications and with the sugar groups of the original antibody.

SEQUENCE LISTING

<110>  Farmahispania S.A.

<120>  Methods for producing homogeneous antibody drug conjugates

<130>  PD10213EP00

<160>  6

<170>  PatentIn version 3.5

<210>  1
<211>  214
<212>  PRT
<213>  Homo sapiens

<400>  1

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Asp Val Asn Thr Ala
            20                  25                  30

Val Ala Trp Tyr Gln Gln Lys Pro Gly Lys Ala Pro Lys Leu Leu Ile
        35                  40                  45

Tyr Ser Ala Ser Phe Leu Tyr Ser Gly Val Pro Ser Arg Phe Ser Gly
    50                  55                  60

Ser Arg Ser Gly Thr Asp Phe Thr Leu Thr Ile Ser Ser Leu Gln Pro
65                  70                  75                  80

Glu Asp Phe Ala Thr Tyr Tyr Cys Gln Gln His Tyr Thr Thr Pro Pro
                85                  90                  95

Thr Phe Gly Gln Gly Thr Lys Val Glu Ile Lys Arg Thr Val Ala Ala
            100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
        180                 185                 190

Ala Cys Glu Val Thr His Gln Gly Leu Ser Ser Pro Val Thr Lys Ser
        195                 200                 205

Phe Asn Arg Gly Glu Cys
    210

<210>  2
<211>  450
<212>  PRT
<213>  Homo sapiens

<400>  2

Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Asn Ile Lys Asp Thr
            20                  25                  30

Tyr Ile His Trp Val Arg Gln Ala Pro Gly Lys Gly Leu Glu Trp Val
        35                  40                  45

Ala Arg Ile Tyr Pro Thr Asn Gly Tyr Thr Arg Tyr Ala Asp Ser Val
    50                  55                  60

Lys Gly Arg Phe Thr Ile Ser Ala Asp Thr Ser Lys Asn Thr Ala Tyr
65                  70                  75                  80

Leu Gln Met Asn Ser Leu Arg Ala Glu Asp Thr Ala Val Tyr Tyr Cys
                85                  90                  95

Ser Arg Trp Gly Gly Asp Gly Phe Tyr Ala Met Asp Tyr Trp Gly Gln
            100                 105                 110

Gly Thr Leu Val Thr Val Ser Ser Ala Ser Thr Lys Gly Pro Ser Val
        115                 120                 125

Phe Pro Leu Ala Pro Ser Ser Lys Ser Thr Ser Gly Gly Thr Ala Ala
    130                 135                 140

Leu Gly Cys Leu Val Lys Asp Tyr Phe Pro Glu Pro Val Thr Val Ser
145                 150                 155                 160

Trp Asn Ser Gly Ala Leu Thr Ser Gly Val His Thr Phe Pro Ala Val
                165                 170                 175

Leu Gln Ser Ser Gly Leu Tyr Ser Leu Ser Ser Val Val Thr Val Pro
        180                 185                 190

Ser Ser Ser Leu Gly Thr Gln Thr Tyr Ile Cys Asn Val Asn His Lys
    195                 200                 205

Pro Ser Asn Thr Lys Val Asp Lys Lys Val Glu Pro Lys Ser Cys Asp
    210                 215                 220

Lys Thr His Thr Cys Pro Pro Cys Pro Ala Pro Glu Leu Leu Gly Gly
225                 230                 235                 240

Pro Ser Val Phe Leu Phe Pro Pro Lys Pro Lys Asp Thr Leu Met Ile
                245                 250                 255

Ser Arg Thr Pro Glu Val Thr Cys Val Val Val Asp Val Ser His Glu
            260                 265                 270

Asp Pro Glu Val Lys Phe Asn Trp Tyr Val Asp Gly Val Glu Val His
        275                 280                 285

Asn Ala Lys Thr Lys Pro Arg Glu Glu Gln Tyr Asn Ser Thr Tyr Arg
    290                 295                 300

Val Val Ser Val Leu Thr Val Leu His Gln Asp Trp Leu Asn Gly Lys
305                 310                 315                 320

Glu Tyr Lys Cys Lys Val Ser Asn Lys Ala Leu Pro Ala Pro Ile Glu
                325                 330                 335

Lys Thr Ile Ser Lys Ala Lys Gly Gln Pro Arg Glu Pro Gln Val Tyr
            340                 345                 350

Thr Leu Pro Pro Ser Arg Glu Glu Met Thr Lys Asn Gln Val Ser Leu
        355                 360                 365

Thr Cys Leu Val Lys Gly Phe Tyr Pro Ser Asp Ile Ala Val Glu Trp
    370                 375                 380

Glu Ser Asn Gly Gln Pro Glu Asn Asn Tyr Lys Thr Thr Pro Pro Val
385                 390                 395                 400

Leu Asp Ser Asp Gly Ser Phe Phe Leu Tyr Ser Lys Leu Thr Val Asp
                405                 410                 415

Lys Ser Arg Trp Gln Gln Gly Asn Val Phe Ser Cys Ser Val Met His
            420                 425                 430

Glu Ala Leu His Asn His Tyr Thr Gln Lys Ser Leu Ser Leu Ser Pro
        435                 440                 445

Gly Lys
    450

<210>  3
<211>  22
<212>  PRT
<213>  Artificial Sequence

<220>
<223>  Signal peptide light chain

<400>  3

Met Asp Met Arg Val Pro Ala Gln Leu Leu Gly Leu Leu Leu Leu Trp
1               5                   10                  15

Leu Ser Gly Ala Arg Cys
            20

<210>  4
<211>  19
<212>  PRT
<213>  Artificial Sequence

<220>
<223>  Signal peptide heavy chain

<400>  4

Met Asp Trp Thr Trp Arg Phe Leu Phe Val Val Ala Ala Ala Thr Gly
1               5                   10                  15

Val Gln Ser

<210>  5
<211>  711
<212>  DNA
<213>  Artificial Sequence

<220>
<223>  Light chain optimized nucleotide sequence

<400>  5
atggacatga gagttcctgc ccagctgctg gggctgcttc tcctgtggct gtctggtgcc      60

cgctgtgaca tccagatgac ccagagcccc tcctccctga gtgccagtgt gggagacaga     120

gtgaccatca cctgcagagc ttctcaggat gtgaacacag ctgtggcctg gtaccagcag     180

aagcctggca aggcccccaa gctgctcatc tactctgcct ccttcctgta cagtggtgtg     240

cccagccgct tctctggaag cagaagtggc acagacttca ccctgaccat ttcttctctt     300

cagcctgaag attttgccac atactactgc cagcagcact acaccacccc tcccaccttt 360

ggccagggaa ccaaagtgga gatcaagagg actgtggctg ctccttctgt cttcatcttc 420

ccacccagtg atgagcaact gaagtctggg acagcctctg tggtgtgcct gctcaacaac 480

ttctacccaa gagaggccaa ggtgcagtgg aaggtggaca atgccctgca gtctggcaac 540

agccaggaga gcgtgacaga gcaggactcc aaggacagca cctacagcct gagcagcaca 600

ctgactctca gcaaggctga ctatgagaag cacaaggtct atgcctgtga ggtgactcac 660

cagggcctgt ccagcccagt caccaagagc ttcaacagag gagagtgtta a 711

<210> 6
<211> 1413
<212> DNA
<213> Artificial Sequence

<220>
<223> Heavy chain optimized nucleotide sequence

<400> 6
atggactgga cctggagatt cctctttgtg gtggctgcag ccacaggtgt ccagagtgaa 60

gtgcagctgg tagagtcagg aggaggtctt gttcagcccg gtggctccct gaggctcagc 120

tgtgctgctt ctggtttcaa catcaaggat acctatatcc actgggtcag gcaggctcct 180

ggcaaaggcc tggaatgggt ggccaggatc taccccacca acggctacac cagatatgct 240

gattctgtca agggccgctt caccatttct gctgacacct ctaagaatac agcctacctg 300

cagatgaaca gtctgagagc tgaggacact gcagtgtact actgcagccg ctggggtgga 360

gatggcttct atgccatgga ttactggggc cagggcactc tggttactgt ttcttctgcc 420

agcacaaagg gtcccagtgt ttttcccctg gcccctagca gcaaaagcac ttctggaggc 480

acagctgccc tgggctgtct ggtgaaagac tacttcccag aacctgtgac tgtctcctgg 540

aacagcggtg ccttgaccag tggagtgcac accttccctg ctgtccttca gagcagtggc 600

ctctactccc tctcctctgt tgtgacagtc ccctcctcca gccttggcac ccagacctac 660

atctgcaatg tcaatcacaa gcccagcaac accaaggtgg ataagaaggt tgagccaccc 720

aagtcctgtg acaaaactca cacatgtcct ccctgcccag ctccagagct gctgggtggt 780

ccttcagtct tcttgtttcc tccaaagcct aaggacacac tgatgatcag caggactcct 840

gaagtcacct gtgtggttgt tgatgtgtct catgaagatc ccgaggtgaa gtttaactgg 900

tatgtggatg gtgtggaagt tcacaatgcc aaaaccaaac caagggagga gcagtacaac 960

tccacataca gagtggtcag tgtgctcaca gtgctgcacc aggactggct gaatggcaag 1020

gagtacaagt gcaaagtgtc caacaaggct ctgcctgccc ccattgagaa gaccatctcc 1080

aaggccaagg gacagccaag agagccccag gtgtatacac ttcctccttc cagagatgag 1140

ctcaccaaga accaggtcag cctgacctgc ctggtcaaag gattctaccc aagtgacatt 1200

gctgtggagt gggagagcaa tgggcagcct gagaacaact acaaaacaac tccacctgtc 1260

ctggacagtg atggaagctt cttcctgtac agcaagctga ctgtggacaa gagcagatgg 1320

cagcagggaa atgtgttctc ctgctctgtg atgcatgagg ccctccacaa ccactacaca 1380

cagaagtcac tgtctctgag ccctgggaag tga 1413

## Description

### FIELD OF THE INVENTION

The present invention refers to the field of medicine and methods of manufacturing, and particularly to methods for producing homogenous antibody conjugates.

### BACKGROUND ART

Cancer is still one of the major threats to human health. However, cancer therapies used today always have more or less adverse side effects to normal tissues. Targeted therapy is a promising strategy to address this challenge. To avoid side effects, drugs should be specifically delivered to cancer cells via binding to ligands that can specifically recognize the cancer-associated biomarkers such as antigens. Among the ligands for targeted therapy, antibodies are excellent candidates because of their specific recognitions and high affinities. Nowadays, antibody-drug conjugates (ADCs) are attracting tremendous attention for targeted cancer therapy.

ADCs are composed of three parts: a monoclonal antibody (mAb) recognizing the target associated antigen over-expressed in specific tumor cells, a cytotoxic agent designed to kill target cancer cells and a chemical linker to attach the cytotoxic agent to the antibody. The antibody binds to specific antigen at the surface of the cancer cell; the whole antibody-drug conjugate is then internalized within the cancer cell, where the linker is degraded and the active drug released. The major advantage of using ADCs relies in the combination of the selectivity of the antibody that acts as the vehicle, and the cell killing ability of cytotoxic drugs. Summarizing, ADCs strategy not only enhances the therapeutic window of potent cytotoxic drugs, but also minimizes chemo-associated side effects.

A report published by Persistence Market Research (PMR) shows that the global market for ADCs is expected to be driven by the advancement in medical technology. The report's authors state that there are 243 antibody-drug conjugates in clinical pipeline for the treatment of cancer. The majority of these agents are investigational drugs in pre-clinical development followed by considerable agents in phase I - III clinical trials (more than 100). Currently 39 clinical studies using ADCs for cancer treatment are active and this number increases up to 114 if the ones under recruitment are considered. These unique targeted drugs comprise both technological and developmental challenges.

Drug Antibody Ratio or DAR is defined as the number of drug molecules per mAb. DAR plays a definitive role in developing ADCs, as it determines the dose needed to produce the desired effect in patients. On the other hand, conjugating too many drug molecules per mAb makes the ADC unstable, toxic and immunogenic; further a high DAR value is associated to higher hydrophobicity and lower solubility which can lead to ADC aggregation. Some experiments point that decreasing the DAR to 2-4 results in a superior therapeutic window.

Regarding the DAR value, ADCs can be classified in two categories: heterogeneous and homogeneous ADCs. Heterogeneous ADCs contain a mix of ADCs with different DAR values; on the opposite, homogeneous ADCs contain only antibodies with a concrete DAR value.

In the category of heterogeneous ADCs, several strategies have been employed for cross-linking the antibody to the drug by a linker using solvent reachable reactive amino acids with nucleophilic groups in antibody side chains. Side chains of lysine (NH₂ group) and cysteine (SH group) have been extensively used for conjugation.

Lysine conjugation is an ADC conjugation method based on connecting the drug to solvent-accessible lysine residues of the antibody. However, there are about 80 lysine residues on a typical antibody and about 10 residues are chemically accessible. Thus, this conjugation modality often gives multiple ADC species with variable DAR and conjugation sites, having every species its own distinct pharmacokinetics and physico-chemical properties. Furthermore, some lysine residues that are critical in antibody-antigen interactions may be modified, resulting in reduced binding affinity. As such, heterogeneous mixtures of ADCs constructed using this conjugation method could potentially lead to a poor therapeutic index. While achievable as seen for instance in the FDA-approved trastuzumab emtansine (Kadcyla®, Genentech) and clinically tested ADC, the lysine-based conjugation requires effort to develop reproducible manufacturing processes ensuring controlled DAR and distribution within a target range (typically 3-4 as a major species).

Cysteines are engaged in interchain and intrachain disulfide bridges in an antibody. In an IgG1 antibody, there are four interchain disulfide bonds, which are more susceptible to reduction than intrachain ones. This allows a controlled reduction of the four interchain disulfide bonds with reductive agents while keeping intrachain disulfide bond intact. This can yield up to eight reactive sulfhydryl groups, facilitating drug conjugation with DAR values of 0-8. Conjugation via cysteine produces more uniform products than lysine conjugation and are also easier to purify and characterize pharmacokinetically. The main problem with these conjugation methods is, as pointed before, the heterogeneous nature of the end products with different DAR values. Despite these drawbacks, cysteine conjugation is a widely utilized and accepted technology in ADCs synthesis. Seattle Genetics' ADC brentuximab vedotin (ADCETRIS®) utilizes this method to conjugate MMAE with the anti-CD30 mAb (cAC10) via an enzymatically cleavable dipeptide linker and thus obtains a heterogeneous commercial ADC.

In order to provide a solution to the drawbacks related to heterogeneous ADCs, different processes for constructing homogeneous ADCs have been developed. Homogeneous ADCs are more desirable from a regulatory perspective as well. Ideally, the final ADC product should exclusively contain an optimally drug-loaded form. Overall, only homogeneous and reproducible ADCs can provide a therapeutic tool that has predictable properties and batch-to-batch consistency. Homogeneous ADCs methods comprise recombinant antibody engineering methods, enzyme mediated methods and linker-based methods, among others.

Recombinant antibody engineering methods consist in adding unique cysteine residues or non-natural amino acids residues into the antibody sequence for site-specific conjugation. These recombinant approaches, however, often require extensive antibody engineering to identify the optimal conjugation sites where unique side chains can be introduced for conjugation, with payloads modified with appropriate linkers. Furthermore, reduction/oxidation protocols are required for each specific mAb. As a result, they are not suitable for converting existing antibodies directly into ADCs. Many of these methods require complex linker and payload modifications that have not been clinically validated and, therefore, present a greater risk for development into therapeutic agents.

Enzyme mediated methods use enzymes to conjugate the linker-drug to the mAb. In order to accomplish a site-specific conjugation, most of the enzyme reactions depend on the existence of a recognition tag in the mAb, which is added by recombinant engineering. These recognition tags can be amino acids, sugar groups or other groups like sialic acid. Like the recombinant antibody engineering methods, these approaches require extensive antibody engineering to identify the optimal sites to add the recognition tags or modifications in the sugar groups. Moreover, these modifications (e.g. sugar groups addition) can affect pharmacokinetic parameters, or cause immunogenic undesired responses.

Many linker-based processes for constructing homogeneous ADCs utilize interchain cysteines for conjugation to obtain homogeneous ADCs with four or eight drugs. The processes are chemically driven and differ from previously discussed processes in that they are focused on linker modifications. As a result, they can be applied to existing antibodies and do not require recombinant antibody re-engineering or unconventional expression systems. One linker-based approach uses hydrophilic linkers, which improves pharmacokinetics and therapeutic index of the ADCs. Another possibility is using bridging linkers which are designed to cross-link antibody interchain cysteines and obtain homogeneous ADCs containing four drugs. Examples of this last approach are using bisulfone linkers, maleimides, or other interchain cross-linkers like dibromopyridazinediones (Jackson DY, 2016). Even these linker-based methods do not relay on mAb recombinant techniques like the other approaches explained previously, the linker chemical design is not trivial, as properties like linker flexibility, geometry or reactivity need to be correctly assessed.

In view of the above, from a therapeutic point of view, homogeneous ADCs are more desirable than heterogeneous ADCs. However, current methods for producing homogeneous ADCs requires time and cost consuming strategies comprising extensive antibody engineering to introduce unique cysteine conjugation sites or enzyme recognition tags, or rational linker chemical design. Thus, there is a clear demand for the construction of ADC platforms that offer homogeneity and direct applicability to the already approved mAbs.

### SUMMARY OF THE INVENTION

One problem to be solved by the present invention may be seen as related to the provision of a method to produce homogeneous ADCs. An additional problem to be solved is related to the provision of ADCs loaded with two different drugs.

The solution is based on the provision of an alternative method to obtain homogeneous ADCs, with the advantage of not requiring antibody sequence engineering and which provide the possibility to obtain ADCs loaded with two different drugs.

Accordingly, a first aspect of the invention relates to a method for producing antibody conjugates having homogeneous payload antibody ratio, the method comprising:
i) providing separate fractions of heavy chains and light chains of an antibody or a fragment thereof both having interchain thiols;
ii) separately conjugating a payload to the interchain thiols of the heavy chains or to the interchain thiols of the light chains or to both; and
iii) assembling the resulting heavy chains with the resulting light chains by mixing them to form antibody conjugates.

Another aspect of the invention relates to a homogeneous composition obtainable by the method as defined in the first aspect, comprising antibody conjugates of the same payload antibody ratio, wherein the antibody in the antibody conjugate has its native amino acid sequence without modifications and with the sugar groups of the original antibody. The two most relevant features of the compositions of the invention compared to the prior art are that all the antibody conjugates within a given composition share the same payload antibody ratio, offering relevant advantages in terms of homogenization of the e.g. pharmaceutical product among different batches, and also in terms of dosing.

The terms used herein have the same meaning as commonly understood by a person skilled in the art. Nevertheless, the term "antibody conjugates" has the same meaning in this description as "antibody drug conjugates" in the art but without limiting the conjugated compound to a drug. Therefore, the term "drug" within the term "antibody drug conjugate" is referred in this description as "payload" to cover all kinds of compounds to be conjugated. Accordingly, the "Drug Antibody Ratio" or "DAR" commonly used in the art, is also referred in this description as "Payload Antibody Ratio". The methods of the invention are applicable to any antibody or a fragment thereof both having interchain thiols, since the conjugation with the payload is performed via interchain thiols reaction. The terms "heavy chain" and "light chain" are abbreviated respectively as HC and LC in this description.

The invention provides a novel platform to generate totally homogeneous DAR ADCs without antibody engineering, that can potentially be applied in particular, to any human IgG1 and IgG3 antibodies, by using conventional approved maleimide linker. Anti-HER2 (lgG1) was used as a model. The method is based on the production of heavy chains and light chains in two recombinant independent cultures, without modification of the original amino acid sequence. Isolated light chains (LC) were effectively conjugated to a drug-linker (MC-vc-PAB-MMAE) construct. Later, mAb assembly was achieved by combining LC-MMAE with isolated heavy chain dimers, in order to obtain a correctly folded non-engineered Cys-based homogeneous ADC. The relevance of the method was validated in terms of ADC homogeneity (HIC-HPLC, MS), purity (SEC-HPLC), isolated antigen recognition (ELISA) and biological activity (HER2-positive breast cancer cells cytotoxicity assays). The present new platform provides an easy way to obtain homogeneous ADCs that could be tested in clinical trials for existing described or approved antibodies to target a disease.

The working examples provided herein demonstrate on one side that the inventors have succeeded in assembling a complete antibody starting with separate fractions of its heavy chains and light chains (see EXAMPLES 2, 3). It is surprising that the light and heavy chains independently produced bind to form a properly folded and functional antibody. And it is even more surprising that a complete antibody is also formed when the light and/or heavy chains have been conjugated with a payload, which a *priori* causes steric impediments and cross-interactions for the correct assembly.

Remarkably, the assembled antibody is fully functional as demonstrated with the cell-based assay and the ELISA assay (EXAMPLE 9 and 2). These two assays demonstrate that the antibody is able to bind to the antigen, as isolated antigen (ELISA) and to the cell surface. On the other side, the inventors have successfully obtained homogenous antibody conjugates following the methods of the invention, which are structurally functional (EXAMPLE 4). Furthermore, the antibody conjugates have shown biological activity (i.e. cytotoxicity) *in vitro* (EXAMPLE 5) and *in vivo,* in mice inoculated with human breast cancer cells (EXAMPLE 6). Therefore, the *in vitro* assembled antibody behaves similarly as its correspondent *in vivo* assembled antibody in terms of isolated antigen binding capacity and biological activity. The methods of the invention have the advantages of not employing antibody-specific cost consuming strategies and not requiring antibody engineering to introduce unique cysteine conjugation sites of conjugation.

By use of the method of the invention is possible to obtain homogenous antibody conjugates with Payload Antibody Ratio 2, conjugating to the light chains or to the Fab interchain thiols of the heavy chains; Payload Antibody Ratio 4, conjugating to both the light chains and to the Fab interchain thiols of the heavy chains, being the payload conjugated to the light chains different or the same as the payload conjugated to the heavy chains; Payload Antibody Ratio 6, conjugating to all the interchain thiols of the heavy chains; and Payload Antibody Ratio 8, conjugating to all the interchain thiols, being the payload conjugated to the light chains different or the same as the payload conjugated to the heavy chains.

Without being bound to the theory, it is believed that this is the first time that a homogeneous composition with antibody conjugates of the same payload antibody ratio is obtained, wherein the antibody has its native amino acid sequence and sugar groups without modifications. As mentioned above, some of the common strategies known in the art require antibody sequence engineering or sugar groups modification, thus, the resulting antibody sequence and/or the sugar groups in the antibody conjugates of the art are in some cases different from the original ones.

### DESCRIPTION OF THE DRAWINGS

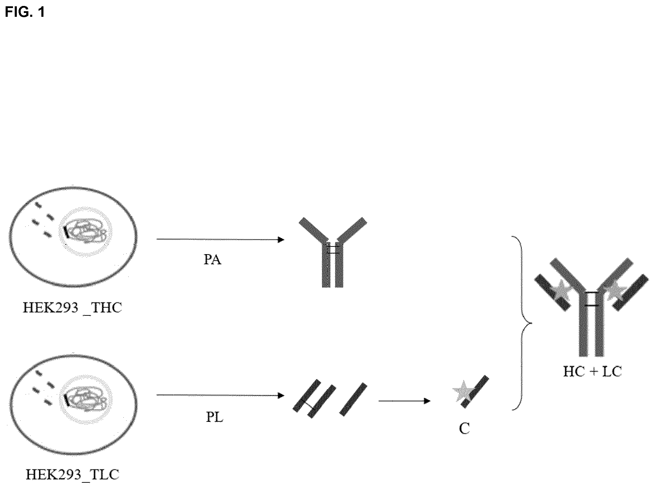

FIG. 1 shows the strategy to obtain DAR 2 homogeneous ADCs. anti-HER2 chains were independently produced in recombinant HEK293 cultures. Then, each chain was purified by affinity chromatography and light chain (LC) was conjugated to MC-vc-PAB-MMAE. Finally, the complete mAb was assembled. PA: Protein A-based affinity purification. PL: Protein L-based affinity purification. C: LC Conjugation. HC+LC: HC+LC assembly.

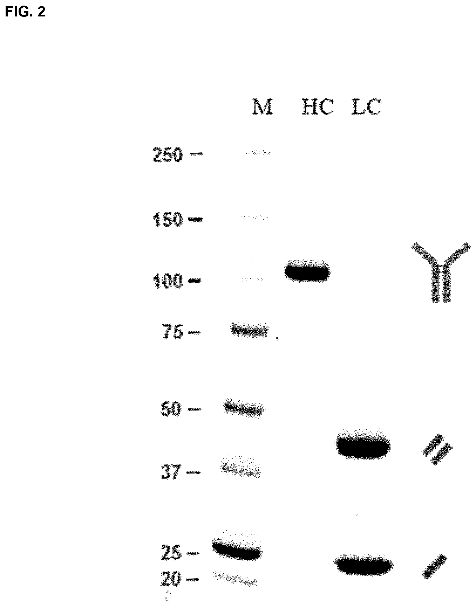

FIG. 2 shows the results of the SDS-PAGE analysis. SDS-PAGE gel on denaturing conditions shows HC and LC conformation of proteins expressed by recombinant HEK293. M: Marker. See working EXAMPLE 1 herein for further details.

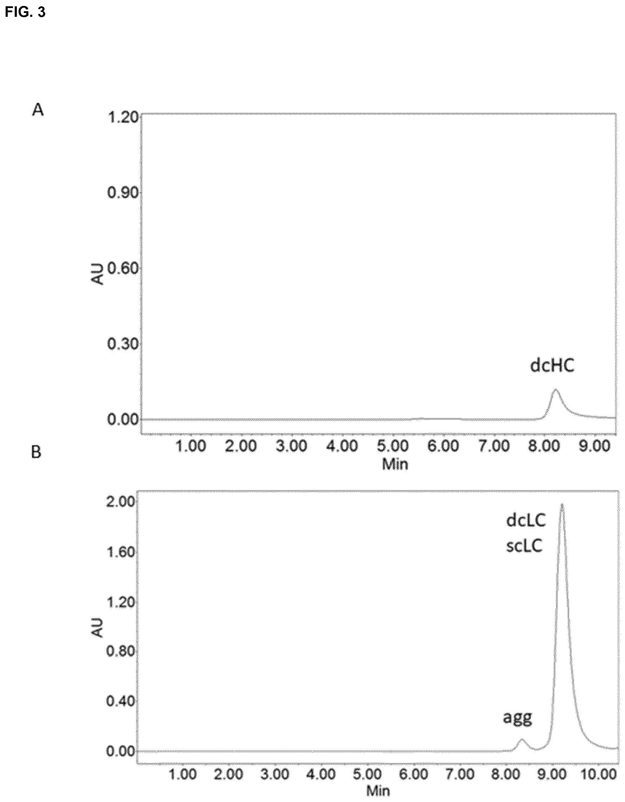

FIG. 3 shows the results of the SEC-HPLC analysis. SEC-HPLC shows (A) HC and (B) LC conformation of protein expressed by recombinant HEK293. AU: UV absorbance at 214 nm. dcHC: double chain HC. scLC: single chain LC. Agg: aggregates. See working EXAMPLE 1 herein for further details.

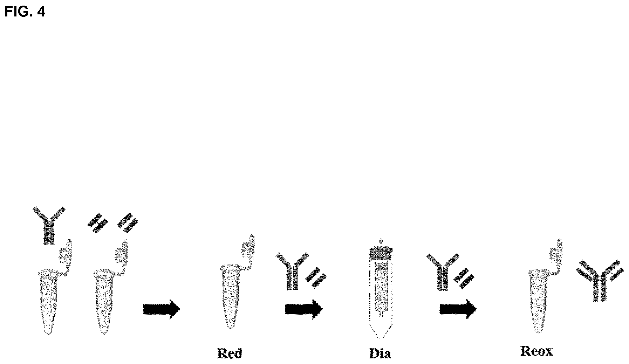

FIG. 4 shows a scheme of the reduction-oxidation assembly (approach 1). Red: reduction step with 16x dcHC DTT 1h 37°C. Dia: diafiltration step with 50 mM citrate buffer pH 6. Reox: reoxidation step with 2x dhAA 3h RT. See working EXAMPLE 2 herein for further details.

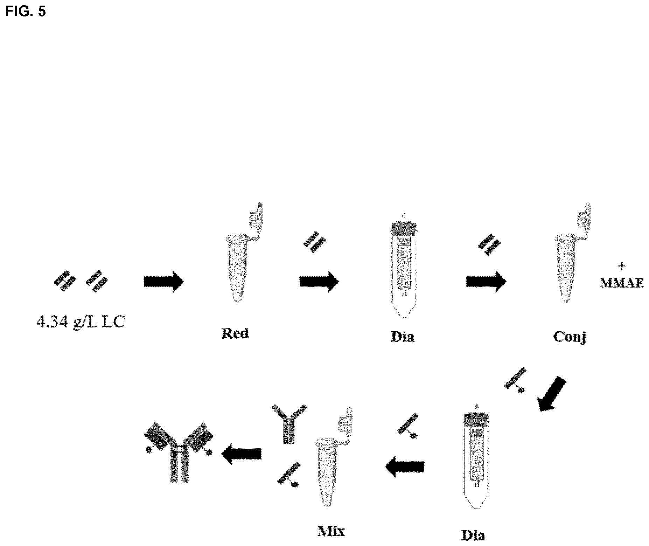

FIG. 5 shows a scheme of the spontaneous assembly (approach 2) to generate homogeneous ADC. Red: reduction step with 100x TCEP 1h 37°C. Dia: diafiltration step with 50 mM citrate pH 6 + DTPA 1mM. Conj: conjugation step with 5x MMAE/free thiol. Dia: diafiltration step with 50 mM citrate pH 6. Mix: mixture step of HC and conjugated LC. See working EXAMPLE 4 herein for further details.

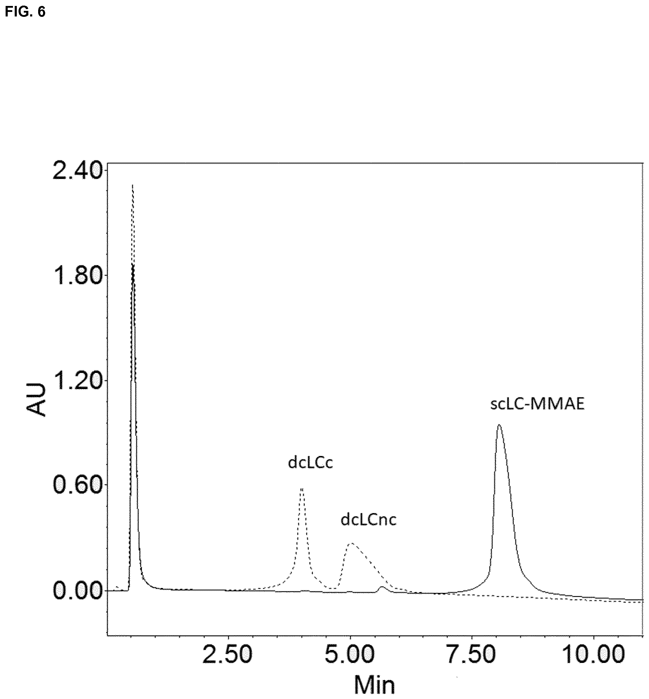

FIG. 6 shows the HIC-HPLC chromatogram. LC (dotted line) appears in two peaks, corresponding to the covalently and un-covalently bonded LC dimers. LC-MMAE (continuous line) appears as a single peak at 8.1 minute. dcLCc: covalently bound double chain LC. dcLCnc: non-covalently bound double chain LC. scLC-MMAE: single chain LC conjugated with MMAE. AU: UV absorbance at 214 nm. See working EXAMPLE 4 herein for further details.

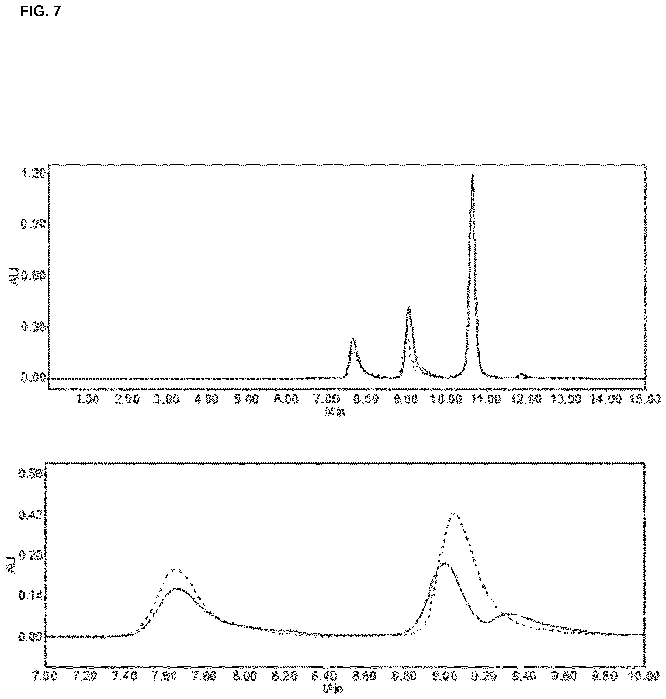

FIG. 7 shows the results of the ADC SEC-HPLC analysis. SEC-HPLC to determine the native structure of the ADC (dotted line) compared to anti-HER2 refolded (continuous line). First peak corresponds to monomer and second peak to LC. Third peak corresponds to sample buffer. (A) show full chromatogram. (B) shows an enlargement of (A) just for monomer and LC. AU: UV absorbance at 214 nm. See working EXAMPLE 4 herein for further details.

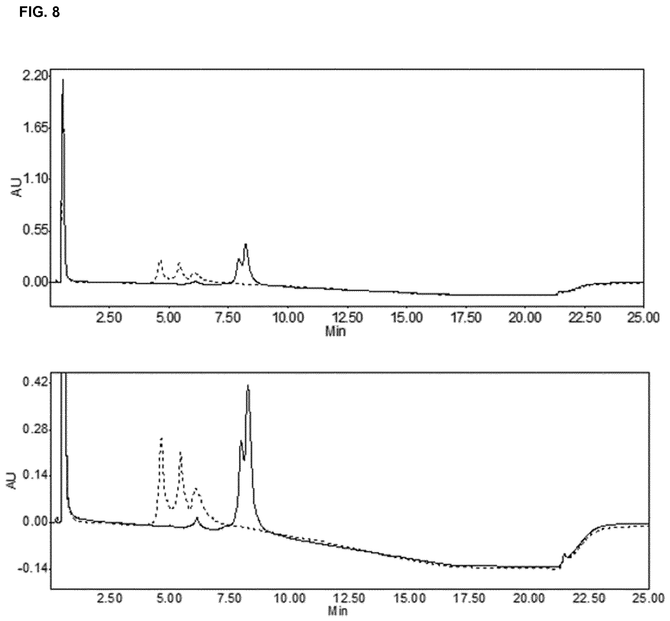

FIG. 8 shows the results of the ADC HIC-HPLC analysis. Dotted line shows unconjugated mAb sample results: First peak is the sample's buffer front, second peak is LC covalently bonded, third peak is monomer mAb and fourth peak is LC un-covalently bonded. Continuous line shows ADC sample results: First peak is the sample's buffer front, second peak is LC un-covalently bonded, third and fourth peak are overlapped, corresponding to monomer ADC and LC-MMAE respectively. AU: UV absorbance at 214 nm. (A) Full chromatogram. (B) Enlargement of (A). See working EXAMPLE 4 herein for further details.

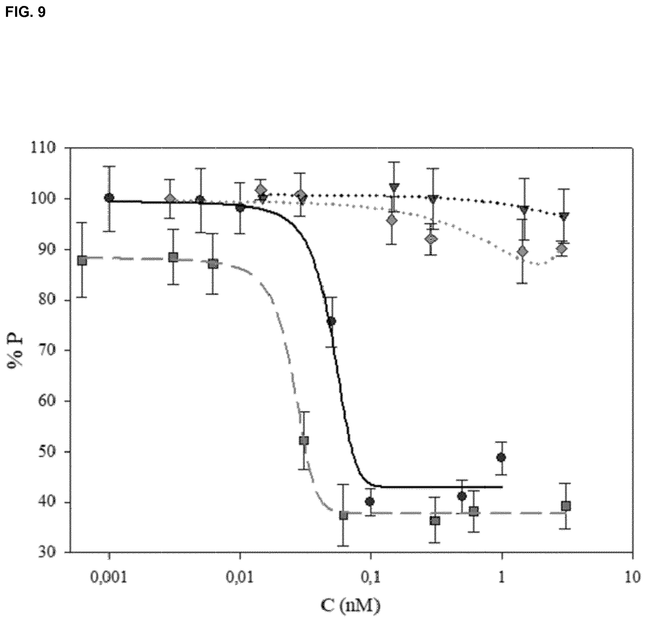

FIG. 9 shows the results of the cell inhibition assay, expressed as % Proliferation (%P) vs Concentration (C) in nM. *Circle*: DAR 2 homogeneous T-MMAE assembled by the strategy described. *Diamond*: assembled non-conjugated mAb by the strategy described. *Square*: DAR 4 heterogeneous T-MMAE (reference). *Inverted triangle*: *in vivo* folded trastuzumab. See working EXAMPLE 5 herein for further details.

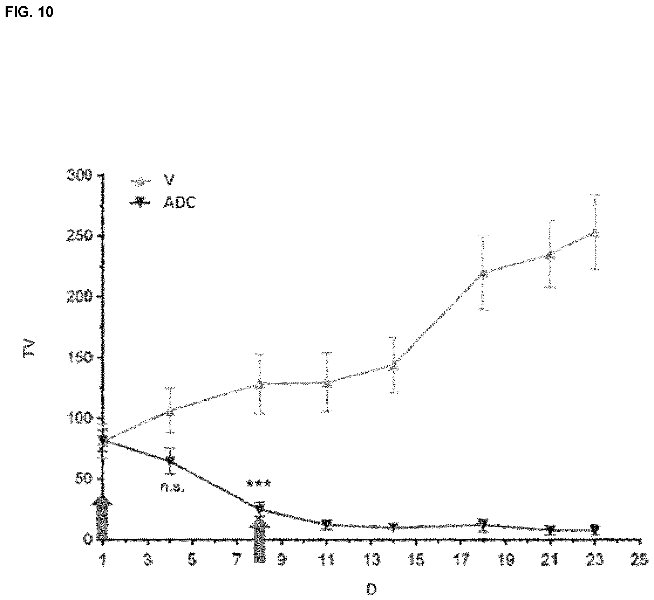

FIG. 10 shows tumor growth of treated mice, expressed as Tumor Volume (TV) in mm³ (Mean ± SEM) vs Day of Treatment (D). Comparative analysis of the primary orthotopic tumor growth of BT-474 cells treated with a vehicle (V) or the ADC. Tumor were monitored twice or three times a week over time. Arrows indicate when treatment (vehicle or ADC) was administered to both groups. Non-measurable tumors are included with value 0. Tumor volumes are not statistically different on day 4 but tumors on ADC treated group have much smaller volumes already on day 8, before the second ADC dose is administered (T-test p = 0.0003). See working EXAMPLE 6 herein for further details.

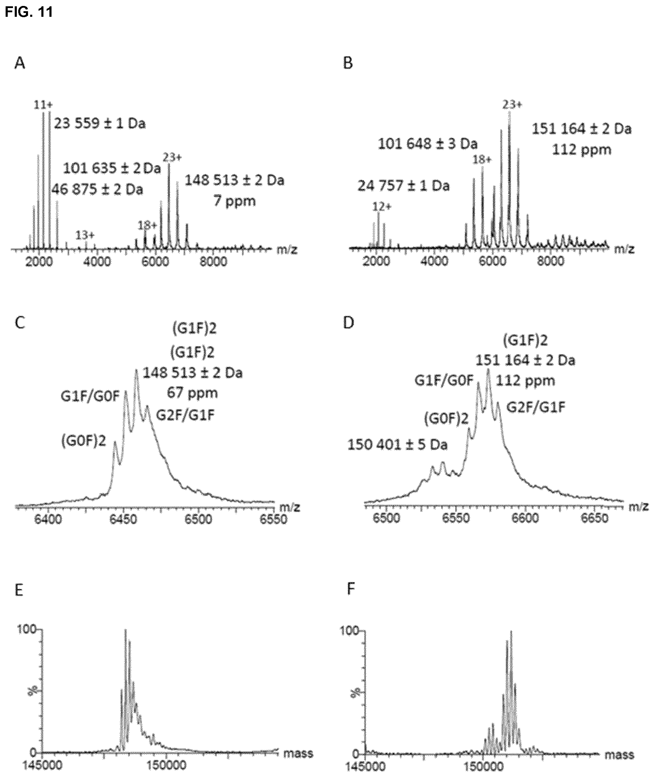

FIG. 11 shows mass spectrometry analysis for mAb and ADC. Full mass spectrum (A: mAb, B: ADC), zoomed spectrum (C: mAb, D: ADC) and deconvoluted spectrum (E: mAb, F: ADC) are shown. Mass increase from mAb to ADC corresponds to drug loading, showing clearly 2 payloads per antibody. Some dissociation is observed for both samples i.e. 100kDa mass detected. No other DAR but DAR 2.0 was observed in the trastuzumab-MC-vc-PAB-MMAE samples. See working EXAMPLE 7 herein for further details.

### DETAILED DESCRIPTION OF THE INVENTION

### Methods for producing antibody conjugates having homogeneous payload antibody ratio

As mentioned above, it is provided a method for producing antibody conjugates having homogeneous payload antibody ratio, the method comprising:
i) providing separate fractions of heavy chains and light chains of an antibody or a fragment thereof both having interchain thiols;
ii) separately conjugating a payload to the interchain thiols of the heavy chains or to the interchain thiols of the light chains or to both; and
iii) assembling the resulting heavy chains with the resulting light chains by mixing them to form antibody conjugates.

### Providing separate fractions of heavy chains and light chains

In a particular embodiment, the step (i) of providing separate fractions of heavy chains and light chains is performed by a method selected from:
a) separately obtaining heavy chains and light chains; and
b) obtaining a complete antibody, separating the heavy chains and the light chains under reduction and denaturalization conditions, and separately refolding the heavy chains and the light chains.

More particularly, the step (i) of providing separate fractions of heavy chains and light chains is performed by separately obtaining heavy chains and light chains. This can be achieved by separate expression of the heavy and light chains in a cell culture or in cell free systems.

In a particular embodiment, separately obtaining heavy chains and light chains is performed by separate expression in cell cultures and optionally purifying. Particularly, the cell cultures are of eukaryotic cells, and particularly mammalian cells such as CHO and HEK cells. In another particular embodiment, the light chains are obtained by expression in prokaryotic cells and optionally purifying.

In another particular embodiment, the provision of separate fractions of heavy chains and light chains is performed by obtaining first a complete antibody by expression of the antibody in e.g. a cell culture, optionally purifying, separating the heavy chains and the light chains under reduction and denaturalization conditions, and separately refolding the heavy chains and the light chains.

In another embodiment, light chains, heavy chains or the complete antibody are obtained in cell free systems (e.g. cell lysate).

In a particular embodiment, the separate fractions of heavy chains and light chains are obtained by a method comprising:
a) separately growing expression cells which comprise a DNA construct with the heavy chain sequence and expression cells which comprise a DNA construct with the light chain sequence;
b) separately expressing the heavy chains and the light chains; and
c) separately purifying the heavy chains and the light chains, comprising solids separation and chromatography, to obtain separate fractions of heavy chains and light chains.

In a particular embodiment, eukaryotic cells such as CHO or HEK cells (e.g. HEK293) are transfected with an expression vector codifying for heavy chains. In a separate culture, cells (eukaryotic or prokaryotic cells, in particular HEK293 cells) are transfected with an expression vector codifying for light chains. Both cell cultures are grown at e.g. 35-38°C and particularly at 37°C. Particularly, cells are cultured at 0.3x10⁶ cell/mL three times per week to keep them in exponential growing phase. The culture medium can be any available for the type of cells but particularly, the culture medium is enriched to optimize the growing phase. Particularly, cells are supplemented with additional medium and expression is induced by antibiotics (puromycin and neomycin). Temperature is kept around 37°C. In this case the expression is constitutive by selective pression, but expression can be induced by any other suitable method/inducer and the expression vector will be designed accordingly.

In a particular embodiment, the expressed heavy chains and light chains are purified. In a particular embodiment, heavy and light chains purification is performed by solids separation with e.g. depth filtration and a bioburden reduction filter plus broth concentration by tangential flow filtration plus chromatography, e.g. affinity, cationic, anionic, molecular exclusion, hydrophobic chromatography; particularly affinity chromatography. In a particular embodiment, heavy chains are purified with protein A affinity chromatography and light chains with protein L affinity chromatography. Solids separation can be performed by e.g. centrifugation, sedimentation, filtration and, particularly is performed by filtration. In a particular embodiment, a buffer can be added to the purified fractions of heavy and light chains, e.g. 50 mM sodium citrate pH 6.

In a more particular embodiment, the DNA construct to express the light chain comprises the expression vector pIRESneo3. More particularly, the DNA codifying for the light chain is inserted between restriction sites Nhel and Agel of this vector. More particularly, the DNA codifying for the light chain is a DNA sequence codifying for the trastuzumab light chain amino acid sequence (e.g. SEQ ID NO: 1). Further, a DNA sequence codifying for an optimized signal peptide for trastuzumab (e.g. SEQ ID NO: 3) is added to the light chain sequence. The corresponding DNA sequences are obtained by a codon optimization method starting with the amino acid sequences. Particularly, the DNA construct to express the light chain comprises the DNA sequence SEQ ID NO: 5.

In another particular embodiment, the DNA construct to express the heavy chain comprises the expression vector pIRESpuro3. More particularly, the DNA codifying for the heavy chain is inserted between restriction sites BamHI and Notl of this vector. More particularly, the DNA codifying for the heavy chain is a DNA sequence codifying for the trastuzumab heavy chain amino acid sequence (e.g. SEQ ID NO: 2). Further, a DNA sequence codifying for an optimized signal peptide for trastuzumab (e.g. SEQ ID NO: 4) is added to the heavy chain sequence. The corresponding DNA sequences are obtained by a codon optimization method starting with the amino acid sequences. Particularly, the DNA construct to express the heavy chain comprises the DNA sequence SEQ ID NO: 6.

### Method to obtain homogenous antibody conjugates with Payload Antibody Ratio 2

By use of the method of the invention is possible to obtain homogenous antibody conjugates with Payload Antibody Ratio 2, conjugating a payload to the light chains. Thus, a particular embodiment of the invention relates to a method for producing antibody conjugates, wherein the resulting antibody conjugates are homogeneous with a payload antibody ratio of 2 and wherein a payload is conjugated to the interchain thiols of the light chains. The method comprises the steps of:
i) providing separate fractions of heavy chains and light chains;
ii) providing light chains with free interchain thiols;
iii) conjugating the payload to the free interchain thiols of the light chains; and
iv) assembling the conjugated light chains obtained in step (iii) with the fraction of unconjugated heavy chains by mixing, to form antibody conjugates.

In a more particular embodiment, the method for producing antibody conjugates having a homogeneous payload antibody ratio of 2 wherein a payload is conjugated to the light chains, comprises the steps of:
i) providing heavy chains and light chains by separate expression in cell cultures and purifying;
ii) providing light chain monomers with free interchain thiols by reducing the light chain fraction with a reducing agent; and in case of excess of reducing agent, eliminating the reducing agent;
iii) conjugating a payload to the free interchain thiols of the light chains;
iv) assembling the conjugated light chains obtained in step (iii) with the fraction of unconjugated heavy chains by mixing the fractions, to form antibody conjugates; and
v) purifying the resulting antibody conjugates.

By use of the method of the invention is possible to obtain homogenous antibody conjugates with Payload Antibody Ratio 2, conjugating a payload to the to the Fab interchain thiols of the heavy chains. Thus, a particular embodiment of the invention relates to a method for producing antibody conjugates, wherein the resulting antibody conjugates are homogeneous with a payload antibody ratio of 2 and wherein a payload is conjugated to the Fab domain interchain thiols of the heavy chains, the method comprising:
i) providing separate fractions of heavy chains and light chains;
ii) providing heavy chain dimers with interchain disulfide bonds between the monomers, and with the Fab domain interchain thiols in free form;
iii) conjugating the payload to the Fab domain interchain thiols in free form of the heavy chain dimers; and
iv) assembling the conjugated heavy chain dimers obtained in step (iii) with the fraction of unconjugated light chains by mixing, to form antibody conjugates.

### Method to obtain homogenous antibody conjugates with Payload Antibody Ratio 4

By use of the method of the invention is possible to obtain homogenous antibody conjugates with Payload Antibody Ratio 4, conjugating a payload to both the light chains and to the Fab interchain thiols of the heavy chains, being the payload conjugated to the light chains different or the same as the payload conjugated to the heavy chains. Thus, a particular embodiment relates to a method for producing antibody conjugates, wherein the resulting antibody conjugates are homogeneous with a payload antibody ratio of 4 and wherein a payload is conjugated to the Fab domain interchain thiols of the heavy chains and a payload is conjugated to the interchain thiols of the light chains, the method comprising:
i) providing separate fractions of heavy chains and light chains;
ii) providing light chains with free interchain thiols;
iii) conjugating a payload to the free interchain thiols of the light chains;
iv) providing heavy chain dimers with interchain disulfide bonds between the monomers, and with the Fab domain interchain thiols in free form;
v) conjugating a payload to the Fab domain interchain thiols in free form of the heavy chain dimers; and
vi) assembling the conjugated heavy chain dimers obtained in step (v) with the conjugated light chains obtained in (iii) by mixing, to form antibody conjugates.

The payload conjugated to the light chains is the same or different from the payload conjugated to the heavy chains.

### Method to obtain homogenous antibody conjugates with Payload Antibody Ratio 6

By use of the method of the invention is possible to obtain homogenous antibody conjugates with Payload Antibody Ratio 6, conjugating a payload to all the interchain thiols of the heavy chains; i.e. the interchain thiols of the Fab domain and the interchain thiols between the heavy chains (in the Fc region). Thus, a particular embodiment relates to a method for producing antibody conjugates, wherein the resulting antibody conjugates are homogeneous with a payload antibody ratio of 6 and wherein a payload is conjugated to the interchain thiols of the heavy chains, the method comprising:
i) providing separate fractions of heavy chains and light chains;
ii) providing heavy chain heavy chains with all interchain thiols in free form;
iii) conjugating a payload to the interchain thiols in free form of the heavy chains; and
iv) assembling the conjugated heavy chain obtained in step (iii) with the fraction of unconjugated light chains by mixing, to form antibody conjugates.

### Method to obtain homogenous antibody conjugates with Payload Antibody Ratio 8

Finally, by use of the method of the invention is possible to obtain homogenous antibody conjugates with Payload Antibody Ratio 8, conjugating to all the interchain thiols, being the payload conjugated to the light chains different or the same as the payload conjugated to the heavy chains. Thus, a particular embodiment relates to a method for producing antibody conjugates, wherein the resulting antibody conjugates are homogeneous with a payload antibody ratio of 8 and wherein a payload is conjugated to interchain thiols of the heavy chains and a payload is conjugated to the interchain thiols of the light chains, the method comprising:
i) providing separate fractions of heavy chains and light chains;
ii) separately providing light chains and heavy chains with all interchain thiols in free form;
iii) conjugating a payload to the interchain thiols in free form of the heavy chains;
iv) conjugating a payload to the free interchain thiols of the light chains; and
v) assembling the conjugated heavy chains obtained in step (iii) with the conjugated light chains obtained in (iv) by mixing, to form antibody conjugates;
wherein the payload conjugated to the light chains is the same or different from the payload conjugated to the heavy chains.

### Provision of light and/or heavy chains with free interchain thiols

In a particular embodiment, the step of providing light chains and/or heavy chains with free interchain thiols, particularly comprises the steps of: (a) reducing the fraction of light chains or heavy chains with a reducing agent; and (b) in case of excess of reducing agent, eliminating the reducing agent. The disulfide bonds between the light chains' dimers are reduced under reduction conditions to obtain light chain monomers with the interchain thiols in free form.

In a particular embodiment, the step of providing heavy chain dimers with interchain disulfide bonds between the monomers and with the Fab domain interchain thiols in free form, comprises the steps of: (a) reducing the fraction of heavy chains with a reducing agent; (b) in case of excess of reducing agent, eliminating the reducing agent; and (c) reoxidizing the resulting fraction of heavy chains.

The reduction step is performed because the cell culture medium that has been used for the expression of the heavy and light chains commonly contains glutathione, which blocks the thiol groups. If a culture medium did not contain glutathione, the reduction step would not be necessary. In a particular embodiment, reduction of the light chains or the heavy chains is performed with tris(2-carboxyethyl)phosphine (TCEP), particularly, with at least 15:1 TCEP, at 25-38°C and at least during 0.5 h. Particularly, 100:1 TCEP is used for 1 h at 37°C. If an excess of reducing agent is used, a step of elimination of the reducing agent is performed, previous to conjugation. This step can be done e.g. by diafiltration with 50 mM citrate pH 6 + DTPA 1 mM.

### Conjugation of the payload to heavy and/or light chains

Conjugation can be performed, e.g. by simply mixing the fraction of heavy/light chains previously treated to have free thiol groups, with the payload in suitable proportions. For instance, free thiols of light chains are conjugated with 5x payload during 1 h at 4°C. As before, a step of diafiltration can be done e.g. with 50 mM citrate pH 6 to eliminate the excess of payload.

### Assembly of the conjugated heavy/light chains to form antibody conjugates

Assembly can be performed, e.g. by simply mixing, in suitable proportions (e.g. 1:1 molar), the fraction of conjugated heavy/light chains, with the complementary part (i.e. the light/heavy chains) to form a complete antibody; that is, depending on the method performed and on the Payload Antibody Ratio, a fraction of conjugated heavy/light chains is assembled with a fraction of unconjugated or conjugated light/heavy chains. The assembly can be performed in physiologic conditions, but a buffer can be added to the mix, e.g. 50 mM citrate buffer at pH 6. Particularly, assembly can be done during 3 h at room temperature.

The resulting antibody conjugates obtained from the methods of the invention can be used directly or can be purified or separated. Thus, in a particular embodiment, the method of the invention further comprises a step of purifying the antibody conjugates. For example, in some embodiments, the antibody conjugates can be separated based on the characteristics of the antibody, the payload and/or the conjugate.

In a particular embodiment, purification is performed by affinity chromatography. As explained before, if there is an excess of (un)conjugated light chains, the product can be purified with Protein A chromatography to capture the conjugated antibody. If the excess is heavy chains, the Protein L chromatography can be used.

### Homogeneous compositions comprising antibody conjugates of the same payload antibody ratio

As discussed before, the invention provides a homogeneous composition obtainable by any of the methods as defined above, comprising antibody conjugates of the same payload antibody ratio, wherein the antibody in the antibody conjugate has its native amino acid sequence without modifications. This means that all the antibody conjugates within the composition share the same payload antibody ratio.

This aspect of the invention is alternatively formulated as homogeneous compositions comprising antibody conjugates of the same payload antibody ratio, wherein the antibody in the antibody conjugate has its native amino acid sequence without modifications.

In a particular embodiment, the homogeneous composition comprises antibody conjugates with a payload antibody ratio of 2 and the payload is conjugated to the Fab domain interchain thiols of the heavy chains or of the light chains. In a more particular embodiment, the antibody conjugates of payload antibody ratio 2 have the payload conjugated to the interchain thiols of the light chains.

In a particular embodiment, the homogeneous composition comprises the antibody conjugates with a payload antibody ratio of 4 and a payload is conjugated to both the Fab domain interchain thiols of the heavy chains and the light chains. In a more particular embodiment, the payload conjugated to the light chains is the same or different from the payload conjugated to the heavy chains.

In another particular embodiment, the homogeneous composition comprises antibody conjugates with a payload antibody ratio of 6 and the payload is conjugated to all interchain thiols of the heavy chains.

In another particular embodiment, the homogeneous composition comprises antibody conjugates with a payload antibody ratio of 8 and a payload is conjugated to all interchain thiols of the heavy chains and a payload is conjugated to the interchain thiols of the light chains. In a more particular embodiment, the payload conjugated to the light chains is the same or different from the payload conjugated to the heavy chains.

To evaluate the technology described, trastuzumab (Herceptin), a monoclonal immunoglobulin G1 (IgG1) targeting HER2 receptor, was selected as a model. This therapeutic antibody has been successfully used in the treatment of HER2 positive breast cancer and is the antibody component of FDA-approved ADC trastuzumab emtansine (Kadcyla). Anticancer drug monometyl auristatin E (MMAE) and valine-citruline linker were selected for their commercial availability and their proven activity in another FDA-approved ADC, brentuximab vedotin. Thus, in a particular embodiment, the antibody is trastuzumab and one payload is monomethyl auristatin E (MMAE) linked via the protease-labile maleimido-caproyl-valine-citrulline-para-amino-benzyloxy carbonyl (MC-vc-PAB) linker. Valine-citrulline is a lysosomal cleavable dipeptide.

### Antibody or a fragment thereof, both having interchain thiols

The term "antibody" as used herein refers to (a) immunoglobulin polypeptides and immunologically active portions of immunoglobulin polypeptides, i.e., polypeptides of the immunoglobulin family, or fragments thereof, that contain an antigen binding site that immunospecifically binds to a specific antigen, or (b) conservatively substituted derivatives of such immunoglobulin polypeptides or fragments that immunospecifically bind to the antigen. The term antibody also includes an "antibody derivative", which means an antibody, as defined above, that is modified by covalent attachment of a heterologous molecule such as, e.g., by attachment of a heterologous polypeptide, or by glycosylation, acetylation or phosphorylation not normally associated with the antibody, and the like.

In a particular embodiment, the antibody is a monoclonal antibody which refers to an antibody that is derived from a single cell clone, including any eukaryotic or prokaryotic cell clone, or a phage clone, and not the method by which it is produced. Thus, the term "monoclonal antibody" as used herein is not limited to antibodies produced through hybridoma technology.

As mentioned, the methods of the invention are applicable to any antibody or a fragment thereof both having interchain thiols, since the conjugation with the payload is performed via interchain thiols reaction.

The term "interchain disulfide bond," in the context of an antibody, refers to a disulfide bond between two heavy chains, or a heavy and a light chain.

The term "interchain thiol" refers to a thiol group of an antibody heavy or light chain that can participate in the formation of an interchain disulfide bond.

The basic unit of an antibody structure is a complex of four polypeptides - two identical low molecular weight ("light") chains and two identical high molecular weight ("heavy") chains, linked together by both non-covalent associations and by disulfide bonds. Different antibodies will have anywhere from one to five of these basic units. The antibody may be represented schematically as a "Y". Each branch of the "Y" is formed by the amino terminal portion of a heavy chain and an associated light chain. The base of the "Y" is formed by the carboxy terminal portions of the two heavy chains. The node of the "Y" is referred to as the hinge region.

Five human antibody classes (IgG, IgA, IgM, IgD and IgE), and within these classes, various subclasses (e.g., IgG1, IgG2, IgG3, IgG4, IgA1 and IgA2) or subclass of immunoglobulin molecule are recognized on the basis of structural differences, such as the number of immunoglobulin units in a single antibody molecule, the disulfide bridge structure of the individual units, and differences in chain length and sequence. The class and subclass of an antibody is its isotype.

The antibody can be an intact antibody or an antigen-binding antibody fragment such as, e.g., a Fab, a F(ab'), a F(ab')2, a Fd chain, a single-chain Fv (scFv), a single-chain antibody, a disulfide-linked Fv (sdFv), a fragment comprising either a VL or VH domain, or fragments produced by a Fab expression library. Antigen-binding antibody fragments, including single-chain antibodies, can comprise the variable region(s) alone or in combination with the entirety or a portion of the following: hinge region, CH1, CH2, CH3, CH4 and CL domains. Also, antigen-binding fragments can comprise any combination of variable region(s) with a hinge region, CH1, CH2, CH3, CH4 and CL domains. In some embodiments, an antibody fragment comprises at least one domain, or part of a domain, that includes interchain disulfide bonds.

Typically, the antibodies are human, rodent (e.g., mouse and rat), donkey, sheep, rabbit, goat, guinea pig, camelid, horse, or chicken. As used herein, "human" antibodies include antibodies having the amino acid sequence of a human immunoglobulin and include antibodies isolated from human immunoglobulin libraries, from human B cells, or from animals transgenic for one or more human immunoglobulin. The antibodies may be monospecific, bispecific, trispecific, or of greater multispecificity.

The antibodies may be directed against antigen of interest, such as medical and/or therapeutic interest. For example, the antigen can be one associated with pathogens (such as but not limited to viruses, bacteria, fungi, and protozoa), parasites, tumor cells, or particular medical conditions. In the case of a tumor-associated antigen (TAA), the cancer may be of the immune system, lung, colon, rectum, breast, ovary, prostate gland, head, neck, bone, or any other anatomical location. Antigens of interest include, but are not limited to, CD30, CD40, Lewis Y, and CD70. In some embodiments, the antigen is CD2, CD20, CD22, CD33, CD38, CD40, CD52, HER2, EGFR, VEGF, CEA, HLA-DR, HLA-Dr10, CA125, CA15-3, CA19-9, L6, Lewis X, alpha fetoprotein, CA 242, placental alkaline phosphatase, prostate specific antigen, prostatic acid phosphatase, epidermal growth factor, MAGE-1, MAGE-2, MAGE-3, MAGE-4, anti-transferrin receptor, p97, MUC1-KLH, gp100, MART1, IL-2 receptor, human chorionic gonadotropin, mucin, P21, MPG, and Neu oncogene product. Some specific useful antibodies include, but are not limited to, BR96 mAb, BR64, mAbs against the CD40 antigen, such as S2C6 mAb, and mAbs against the CD30 antigen, such as AC10. Many other internalizing antibodies that bind to tumor specific antigens can be used.

The term "tumor-specific antigen" as used herein will be understood to connote an antigen characteristic of a particular tumor, or strongly correlated with such a tumor. However, tumor-specific antigens are not necessarily unique to tumor tissue, however, i.e., that antibodies to them may cross-react with antigens of normal tissue. Where a tumor-specific antigen is not unique to tumor cells, it frequently occurs that, as a practical matter, antibodies binding to tumor-specific antigens are sufficiently specific to tumor cells to carry out the desired procedures without unwarranted risk or interference due to cross-reactions. Many factors contribute to this practical specificity. For example, the amount of antigen on the tumor cell may greatly exceed the amount of the cross-reactive antigen found on normal cells, or the antigen on the tumor cells may be more effectively presented. Therefore, the term "tumor-specific antigen" relates herein to a specificity of practical utility, and is not intended to denote absolute specificity or to imply an antigen is unique to the tumor.

The antibody may be a polyclonal antibody or a monoclonal antibody. When the subject is a human subject, the antibody may be obtained by immunizing any animal capable of mounting a usable immune response to the antigen. The animal may be a mouse, rat, goat, sheep, rabbit or other suitable experimental animal. The antigen may be presented in the form of a naturally occurring immunogen, or a synthetic immunogenic conjugate of a hapten and an immunogenic carrier. In the case of a monoclonal antibody, antibody producing cells of the immunized animal may be fused with "immortal" or "immortalized" human or animal cells to obtain a hybridoma which produces the antibody. If desired, the genes encoding one or more of the immunoglobulin chains may be cloned so that the antibody may be produced in different host cells, and if desired, the genes may be mutated so as to alter the sequence and hence the immunological characteristics of the antibody produced. Human monoclonal antibodies may be made by any of numerous techniques known in the art.

The antibody can be, e.g., a murine, a chimeric, humanized, or fully human antibody produced by techniques well-known to one of skill in the art. Recombinant antibodies, such as chimeric and humanized monoclonal antibodies, comprising both human and non-human portions, which can be made using standard recombinant DNA techniques, are useful antibodies. A chimeric antibody is a molecule in which different portions are derived from different animal species, such as those having a variable region derived from a murine monoclonal and human immunoglobulin constant regions. Humanized antibodies are antibody molecules from non-human species having one or more complementarity determining regions (CDRs) from the non-human species and a framework region from a human immunoglobulin molecule. Such chimeric and humanized monoclonal antibodies can be produced by recombinant DNA techniques known in the art.

Completely human antibodies can be produced, e.g., using transgenic mice that are incapable of expressing endogenous immunoglobulin heavy and light chains genes, but which can express human heavy and light chain genes. The transgenic mice are immunized in the normal fashion with a selected antigen or a portion thereof. Monoclonal antibodies directed against the antigen can be obtained using conventional hybridoma technology. The human immunoglobulin transgenes harbored by the transgenic mice rearrange during B cell differentiation, and subsequently undergo class switching and somatic mutation. Thus, using such a technique, it is possible to produce therapeutically useful IgG, IgA, IgM and IgE antibodies.

Human(ized) IgG1 represents the majority IgG subclass of the current mAbs approved by FDA. Thus, in a particular embodiment, the antibody is a human IgG, particularly an IgG1, and more particularly a human(ized) IgG1.

### Payload

A payload is generally understood as any compound, molecule or structure, which is conjugated to the antibody. In a particular embodiment, the payload is selected from the group consisting of a drug, an oligonucleotide, a peptide, an enzyme, a protein, a substrate, an inorganic compound, a chelate, a radioactive agent, a detectable reagent, and a nanoparticle.

The term "drug" as used herein means an element, compound, agent, or molecular entity, including, e.g., a pharmaceutical, therapeutic, or pharmacologic compound. Drugs can be natural or synthetic or a combination thereof; they can be small molecules, oligonucleotides, peptides, enzymes, and proteins. Drugs can be a cytostatic agent or cytotoxic agent, an immunosuppressive agent, a toxin, a chelate, a compound, a molecule and the like. A "therapeutic drug" is an agent that exerts a therapeutic (e.g., beneficial) effect on target cells, e.g. cancer cells or immune cells (e.g., activated immune cells), either alone or in combination with another agent (e.g., a prodrug converting enzyme in combination with a prodrug). Typically, therapeutic drugs useful in accordance with the methods and compositions described herein are those that exert a cytotoxic, cytostatic, or immunosuppressive effect.

"Cytotoxic agent," in reference to the effect of an agent on a cell, means killing of the cell. "Cytostatic agent" means an inhibition of cell proliferation. Cytotoxic and cytostatic drugs include antibiotics (e.g., adriamycin), antitumor agents such as auristatins and auristatin derivatives, methotrexate, mitomycin C, daunorubicin, doxorubicin, and vinblastine, 5-fluorouracil DNA minor grove binders, DNA replication inhibitors, alkylating agents (e.g., platinum complexes such as cisplatin, mono(platinum), bis(platinum) and tri-nuclear platinum complexes and carboplatin), antiparasitic agents (e.g., pentamidine isethionate), anthracyclines, antifolates, antimetabolites, chemotherapy sensitizers, duocarmycins, etoposides, fluorinated pyrimidines, ionophores, lexitropsins, nitro-soureas, platinols, pre-forming compounds, purine antimetabolites, puromycins, radiation sensitizers, steroids, taxanes, topoisomerase inhibitors, vinca alkaloids, antimicrobial agents, antimicrotubule agents, or the like. When an antibody is conjugated to such a drug, it serves to direct the drug to the sites where the corresponding antigen occurs.

Cytotoxic or cytostatic agents include, e.g., an androgen, anthramycin (AMC), asparaginase, 5-azacytidine, azathioprine, bleomycin, busulfan, buthionine sulfoximine, camptothecin, carboplatin, carmustine (BSNU), CC-1065, chlorambucil, cisplatin, colchicine, cyclophosphamide, cytarabine, cytidine arabinoside, cytochalasin B, dacarbazine, dactinomycin (formerly actinomycin), daunorubicin, decarbazine, docetaxel, doxorubicin, an estrogen, 5-fluordeoxyuridine, 5-fluorouracil, gramicidin D, hydroxyurea, idarubicin, ifosfamide, irinotecan, lomustine (CCNU), mechlorethamine, melphalan, 6-mercaptopurine, methotrexate, mithramycin, mitomycin C, mitoxantrone, nitroimidazole, paclitaxel, plicamycin, procarbizine, streptozotocin, tenoposide, 6-thioguanine, thioTEPA, topotecan, vinblastine, vincristine, vinorelbine, VP-16 and VM-26.

Other cytotoxic agents include, e.g., dolastatins, DNA minor groove binders such as the enediynes (e.g., calicheamicin) and lexitropsins, duocarmycins, taxanes (e.g., paclitaxel and docetaxel), puromycins, CC-1065, SN-38, topotecan, morpholino-doxorubicin, rhizoxin, cyanomorpholinodoxorubicin, echinomycin, combretastatin, netropsin, epothilone A, B or D, estramustine, cryptophysins, cemadotin, maytansinoids, discodermolide, eleutherobin, and mitoxantrone.

In certain embodiments, the cytotoxic agent is a chemotherapeutic such as, e.g., doxorubicin, melphalan, vinca alkaloids, methotrexate, mitomycin C or etoposide. In addition, potent agents such as CC-1065 analogues, calicheamicin, maytansine, analogues of dolastatin 10, rhizoxin, and palytoxin can be linked to proteins.

In particular embodiments, the cytotoxic or cytostatic agent is auristatin E (also known in the art as dolastatin-10) or a derivative thereof. Typically, the auristatin E derivative is, e.g., an ester formed between auristatin E and a keto acid. For example, auristatin E can be reacted with paraacetyl benzoic acid or benzoylvaleric acid to produce AEB and AEVB, respectively. Other typical auristatin derivatives include AFP, MMAF, and MMAE. In a particular embodiment, the payload is monomethyl auristatin E (MMAE) linked to the antibody via the protease-labile maleimido-caproyl-valine-citrulline-para-amino-benzyloxy carbonyl (MC-vc-PAB) linker.

In certain embodiments, the cytotoxic or cytostatic agent is an anti-tubulin agent. Examples of anti-tubulin agents include, but are not limited to, taxanes (e.g., Taxol® (paclitaxel), Taxotere® (docetaxel)), T67 (Tularik), vinca alkyloids (e.g., vincristine, vinblastine, vindesine, and vinorelbine), and dolastatins (e.g., auristatin E, AFP, MMAF, MMAE, AEB, AEVB). Other antitubulin agents include, e.g., baccatin derivatives, taxane analogs (e.g., epothilone A and B), nocodazole, colchicine and colcimid, estramustine, cryptophysins, cemadotin, maytansinoids, combretastatins, discodermolide, and eleutherobin.

In some embodiments, the cytotoxic or immunosuppressive agent is an antimetabolite. The antimetabolite can be, e.g., a purine antagonist (e.g., azothioprine or mycophenolate mofetil), a dihydrofolate reductase inhibitor (e.g., methotrexate), acyclovir, gangcyclovir, zidovudine, vidarabine, ribavarin, azidothymidine, cytidine arabinoside, amantadine, dideoxyuridine, iododeoxyuridine, poscarnet, or trifluridine.

In other embodiments, the cytotoxic or immunosuppressive agent is tacrolimus, cyclosporine or rapamycin. In further embodiments, the cytotoxic agent is aldesleukin, alemtuzumab, alitretinoin, allopurinol, altretamine, amifostine, anastrozole, arsenic trioxide, bexarotene, bexarotene, calusterone, capecitabine, celecoxib, cladribine, Darbepoetin alfa, Denileukin diftitox, dexrazoxane, dromostanolone propionate, epirubicin, Epoetin alfa, estramustine, exemestane, Filgrastim, floxuridine, fludarabine, fulvestrant, gemcitabine, goserelin, idarubicin, ifosfamide, imatinib mesylate, Interferon alfa-2a, irinotecan, letrozole, leucovorin, levamisole, meclorethamine or nitrogen mustard, megestrol, mesna, methotrexate, methoxsalen, mitomycin C, mitotane, nandrolone phenpropionate, oprelvekin, oxaliplatin, pamidronate, pegademase, pegaspargase, pegfilgrastim, pentostatin, pipobroman, plicamycin, porfimer sodium, procarbazine, quinacrine, rasburicase, Sargramostim, streptozocin, tamoxifen, temozolomide, teniposide, testolactone, thioguanine, toremifene, tretinoin, uracil mustard, valrubicin, vinblastine, vincristine, vinorelbine and zoledronate.

In some embodiments, the agent is an immunosuppressive agent. The immunosuppressive agent can be, e.g., gancyclovir, tacrolimus, cyclosporine, rapamycin, cyclophosphamide, azathioprine, mycophenolate mofetil or methotrexate. Alternatively, the immunosuppressive agent can be, e.g., a glucocorticoid (e.g., cortisol or aldosterone) or a glucocorticoid analogue (e.g., prednisone or dexamethasone).

In some embodiments, the immunosuppressive agent is an anti-inflammatory agent, such as arylcarboxylic derivatives, pyrazole-containing derivatives, oxicam derivatives and nicotinic acid derivatives. Classes of anti-inflammatory agents include, e.g., cyclooxygenase inhibitors, 5-lipoxygenase inhibitors, and leukotriene receptor antagonists. Suitable cyclooxygenase inhibitors include meclofenamic acid, mefenamic acid, carprofen, diclofenac, diflunisal, fenbufen, fenoprofen, ibuprofen, indomethacin, ketoprofen, nabumetone, naproxen, sulindac, tenoxicam, tolmetin, and acetylsalicylic acid. Suitable lipoxygenase inhibitors include redox inhibitors (e.g., catechol butane derivatives, nordihydroguaiaretic acid (NDGA), masoprocol, phenidone, lanopalen, indazolinones, naphazatrom, benzofuranol, alkylhydroxylamine), and non-redox inhibitors (e.g., hydroxythiazoles, methoxyalkylthiazoles, benzopyrans and derivatives thereof, methoxytetrahydropyran, boswellic acids and acetylated derivatives of boswellic acids, and quinolinemethoxyphenylacetic acids substituted with cycloalkyl radicals), and precursors of redox inhibitors.

Other suitable lipoxygenase inhibitors include antioxidants (e.g., phenols, propyl gallate, flavonoids and/or naturally occurring substrates containing flavonoids, hydroxylated derivatives of the flavones, flavonol, dihydroquercetin, luteolin, galangin, orobol, derivatives of chalcone, 4,2',4'-trihydroxychalcone, ortho-aminophenols, N-hydroxyureas, benzofuranols, ebselen and species that increase the activity of the reducing selenoenzymes), iron chelating agents (e.g., hydroxamic acids and derivatives thereof, N-hydroxyureas, 2-benzyl-1-naphthol, catechols, hydroxylamines, carnosol trolox C, catechol, naphthol, sulfasalazine, zyleuton, 5-hydroxyanthranilic acid and 4-(omega-arylalkyl)phenylalkanoic acids), imidazole-containing compounds (e.g., ketoconazole and itraconazole), phenothiazines, and benzopyran derivatives.

Yet other suitable lipoxygenase inhibitors include inhibitors of eicosanoids (e.g., octadecatetraenoic, eicosatetraenoic, docosapentaenoic, eicosahexaenoic and docosahexaenoic acids and esters thereof, PGE1 (prostaglandin E1), PGA2 (prostaglandin A2), viprostol, 15-monohydroxyeicosatetraenoic, 15-monohydroxy-eicosatrienoic and 15-monohydroxyeicosapentaenoic acids, and leukotrienes B5, C5 and D5), compounds interfering with calcium flows, phenothiazines, diphenylbutylamines, verapamil, fuscoside, curcumin, chlorogenic acid, caffeic acid, 5,8,11,14-eicosatetrayenoic acid (ETYA), hydroxyphenylretinamide, lonapalen, esculin, diethylcarbamazine, phenantroline, baicalein, proxicromil, thioethers, diallyl sulfide and di-(1-propenyl) sulfide.

In particular embodiments, the payload is a drug known for the treatment of an autoimmune disease. The anti-autoimmune disease agent can include, but is not limited to, the following: cyclosporine, cyclosporine A, mycophenylate mofetil, sirolimus, tacrolimus, enanercept, prednisone, azathioprine, methotrexate, cyclophosphamide, aminocaproic acid, chloroquine, hydroxychloroquine, hydrocortisone, dexamethasone, chlorambucil, DHEA, danazol, bromocriptine, meloxicam and infliximab.

In particular embodiments, the payload is therapeutic agent that is an anti-infectious disease agent. The anti-infectious disease agent can be, but not limited to, the following: β-lactam antibiotics, such as penicillin G, penicillin V, cloxacilliin, dicloxacillin, methicillin, nafcillin, oxacillin, ampicillin, amoxicillin, bacampicillin, azlocillin, carbenicillin, mezlocillin, piperacillin and ticarcillin; aminoglycosides such as amikacin, gentamicin, kanamycin, neomycin, netilmicin, streptomycin and tobramycin; macrolides such as azithromycin, clarithromycin, erythromycin, lincomycin and clindamycin; tetracyclines such as demeclocycline, doxycycline, minocycline, oxytetracycline and tetracycline; quinolones such as cinoxacin, and nalidixic acid; fluoroquinolones such as ciprofloxacin, enoxacin, grepafloxacin, levofloxacin, lomefloxacin, norfloxacin, ofloxacin, sparfloxacin and trovafloxicin; polypeptides such as bacitracin, colistin and polymyxin B; sulfonamides such as sulfisoxazole, sulfamethoxazole, sulfadiazine, sulfamethizole and sulfacetamide; and other antibacterial agents, such as trimethoprim, sulfamethazole, chloramphenicol, vancomycin, metronidazole, quinupristin, dalfopristin, rifampin, spectinomycin and nitrofurantoin; and antiviral agents, such as general antiviral agents such as idoxuradine, vidarabine, trifluridine, acyclovir, famcicyclovir, pencicyclovir, valacyclovir, gancicyclovir, foscarnet, ribavirin, amantadine, rimantadine, cidofovir; antisense oligonucleotides, immunoglobulins and interferons; and drugs for HIV infection such as tenofovir, emtricitabine, zidovudine, didanosine, zalcitabine, stavudine, lamivudine, nevirapine, delavirdine, saquinavir, ritonavir, indinavir and nelfinavir.

Toxins are usefully conjugated to antibodies specific for antigens associated with tumor, parasite or microbial cells. The toxin may be from, e.g., a plant (e.g., ricin or abrin), animal (e.g., a snake venom), or microbial (e.g., diphtheria or tetanus toxin).

In certain embodiments, the payload is an oligonucleotide, a peptide, an enzyme, or a protein, all having the common meaning in the art.

Particular drugs of interest for their relevant therapeutic applications are, e.g. Calicheamicins, MMAE, MMAF, SN-38 (analog of camptothecin - a topoisomerase I inhibitor, which is the active metabolite of irinotecan), PBD Dimers, Mertansine/emtansine (DM1), ravtansine/soravtansine (DM4), Doxorubicin, PE38 (*Pseudomonas* exotoxin A), Gemcitabine, Duocarmycins, Amberstatin 269.

In other embodiments, the payload is a detectable reagent, to be used e.g. in diagnostics, imaging, identification of target cells and other uses. Particularly, a detectable reagent is a labelled compound such as a radioactive element, or a substrate.

In other embodiments, the payload is a nanoparticle, including metallic and rare earth elements nanoparticles.

### Conjugation of the payload to antibody. Linkers

The payload to be conjugated to the antibody can be previously derivatized or modified to be able to react with the antibody interchain thiols. Examples of modification and derivatization are chemical reactions or addition of a linker with thiols.

The payload can be linked to the antibody by a linker. A cleavable linker is typically susceptible to cleavage under intracellular conditions. Suitable cleavable linkers include, e.g., a peptide linker cleavable by an intracellular protease, such as lysosomal protease or an endosomal protease. In exemplary embodiments, the linker can be a dipeptide linker, such as a valine-citrulline (val-cit) or a phenylalanine-lysine (phe-lys) linker. Other suitable linkers include linkers hydrolyzable at a pH of less than 5.5, such as a hydrazone linker. Additional suitable cleavable linkers include disulfide linkers. The linker includes a group for linkage to the antibody. In a particular embodiment, the linker includes sulfhydryl reactive groups (e.g., malemide, haloacetamides (e.g., iodo, bromo or chloro). The linker unit is typically an amino acid unit, such as e.g. a dipeptide, tripeptide, tetrapeptide, pentapeptide, hexapeptide, heptapeptide, octapeptide, nonapeptide, decapeptide, undecapeptide or dodecapeptide unit. The linker unit can be cleavage or non-cleavable inside the cell.

A spacer unit, if present, links a linker unit to the drug, alternatively, a spacer unit can link a stretcher unit to a drug moiety when the linker unit is absent. The spacer unit can also link a drug to an antibody when both the linker unit and stretcher unit are absent.

Suitable methods for introducing sulfhydryl groups in the payload include chemical means (e.g., malemide, haloacetamides (e.g., iodo, bromo or chloro), haloesters (e.g., iodo, bromo or chloro), halomethyl ketones (e.g., iodo, bromo or chloro), benzylic halides (e.g., iodide, bromide or chloride), vinyl sulfone and pyridyithio).

### Payload antibody ratio

Different analytical methods can be used to determine the payload antibody ratio and the location of the drugs on the antibody, e.g. Hydrophobic interaction chromatography-high performance liquid chromatography (HIC-HPLC), Liquid chromatography-mass spectrometry (LC-MS) and Reversed phase HPLC (RP-HPLC). Another embodiment of an analytical tool is chromatography on a polystyrene-divinylbenzene reversed-phase (PLRP) column; the column support is composed of crosslinked divinylbenzene, rather than a typical reversed phase column built on a silica support which can nonspecifically retain proteins.

Various analytical methods can be used to determine the yields of the antibody conjugates. For example, in one embodiment Hydrophobic interaction chromatography (HIC) is the analytical method used to determine yields.

### Applications of the antibody conjugates

### Immunodiagnosis

In one embodiment, an antibody is conjugated to a detectable label for use in *in vitro* immunodiagnosis. The label may be a radiolabel, fluorophore, or enzyme, which is directly or indirectly conjugatable to antibody. The sample may be of clinical (e.g., blood, urine, semen, or cerebrospinal fluid, or a solid tissue or organ) or non-clinical (e.g., soil, water, food) nature. The assay may be qualitative or quantitative, and in any desired format, including sandwich and competitive formats.

### Immunoimaging

An antibody conjugate may also be used for *in vivo* immunoimaging. For this purpose, the antibody is labeled by means, which permit external visualization of its position or location within a subject or part thereof, such as an organ. Typically, an immunoimaging agent will be an antibody labeled directly (as with Technetium) or indirectly (as with chelated Indium) with a suitable radioisotope. After injection into the patient, the location of the conjugate may be tracked by a detector sensitive to particles emitted by the radiolabel, e.g., a gamma-scintillation camera in the case of a gamma emitter.

### Therapeutic applications

For immunotherapy, an antibody can be conjugated to suitable drug, such as a cytotoxic or cytostatic agent, an immunosuppressive agent, a radioisotope, a toxin, or the like. The conjugate can be used for inhibiting the multiplication of a tumor cell or cancer cell, causing apoptosis in a tumor or cancer cell, or for treating cancer in a patient. The conjugate can be used accordingly in a variety of settings for the treatment of animal cancers. The conjugate can be used to deliver a drug to a tumor cell or cancer cell. Without being bound by theory, in some embodiments, the conjugate binds to or associates with a cancer-cell or a tumor-associated antigen, and the conjugate and/or drug can be taken up inside a tumor cell or cancer cell through receptor-mediated endocytosis.

The antigen can be attached to a tumor cell or cancer cell or can be an extracellular matrix protein associated with the tumor cell or cancer cell. Once inside the cell, one or more specific peptide sequences within the conjugate (e.g., in a linker) are hydrolytically cleaved by one or more tumor-cell or cancer-cell-associated proteases, resulting in release of the drug. The released drug is then free to migrate within the cell and induce cytotoxic or cytostatic or other activities. In some embodiments, the drug is cleaved from the antibody outside the tumor cell or cancer cell, and the drug subsequently penetrates the cell, or acts at the cell surface.

Thus, in some embodiments, the conjugate binds to the tumor cell or cancer cell. In some embodiments, the conjugate binds to a tumor cell or cancer cell antigen, which is on the surface of the tumor cell or cancer cell. In other embodiments, the conjugate binds to a tumor cell or cancer cell antigen, which is an extracellular matrix protein, associated with the tumor cell or cancer cell.

In other embodiments, methods for treating or preventing cancer are provided, including administering to a patient in need thereof an effective amount of a conjugate and a chemotherapeutic agent. In some embodiments, the chemotherapeutic agent is that with which treatment of the cancer has not been found to be refractory. In some embodiments, the chemotherapeutic agent is that with which the treatment of cancer has been found to be refractory. The conjugate can be administered to a patient that has also undergone a treatment, such as surgery for treatment for the cancer. In another embodiment, the additional method of treatment is radiation therapy.

The antibody conjugate can also be used in an *in vitro* or *ex vivo* fashion, such as for the treatment of certain cancers, including, but not limited to leukemias and lymphomas, such treatment involving autologous stem cell transplants. This can involve a multi-step process in which the animal's autologous hematopoietic stem cells are harvested and purged of all cancer cells, the animal's remaining bone-marrow cell population is then eradicated via the administration of a high dose of a conjugate with or without accompanying high dose radiation therapy, and the stem cell graft is infused back into the animal. Supportive care is then provided while bone marrow function is restored and the animal recovers.

The conjugates are useful for killing or inhibiting the replication of a cell that produces an autoimmune disease or for treating an autoimmune disease. The conjugates can be used accordingly in a variety of settings for the treatment of an autoimmune disease in a patient. The conjugates can be used to deliver a drug to a target cell.

The conjugates are useful for killing or inhibiting the multiplication of a cell that produces an infectious disease or for treating an infectious disease. The conjugates can be used accordingly in a variety of settings for the treatment of an infectious disease in a patient. The ADCs can be used to deliver a drug to a target cell. In one embodiment, the antibody binds to the infectious disease cell. In some embodiments, the conjugate kills or inhibit the multiplication of cells that produce a particular infectious disease.

Therefore, another aspect of the invention relates to the homogeneous compositions comprising the antibody conjugates obtainable by the methods of the invention for use as medicaments. Furthermore, other aspects of the invention relate to the homogeneous compositions for use in immunotherapy, particularly in the treatment and/or prevention of diseases or conditions, and more particularly of the diseases mentioned before, such as cancer, autoimmune diseases, and infectious diseases. Alternatively, other aspects of the invention relate to methods for treating or preventing diseases and conditions, particularly, the diseases mentioned before, including administering to a patient in need thereof an effective amount of the homogeneous compositions comprising the antibody conjugates according to the invention.

### Multi-Drug Therapy

The method of the invention allows conjugating the antibody with two different payloads, thus being interesting for a multi-drug cell targeted therapy; i.e. since the two drugs are conjugated to the same antibody, both are targeted together to the site of effect.

### Pharmaceutical compositions

An aspect of the invention relates to a pharmaceutical composition comprising the homogeneous antibody conjugates according to the invention. The pharmaceutical compositions can be in any form that allows for the composition to be administered to a patient. It is not the objective of this description to provide details of the formulations, excipients, administration forms, doses, etc that are commonly known by the skilled in the art.

Unless otherwise defined, all technical and scientific terms used herein have the same meaning as commonly understood by a person skilled in the art. Methods and materials similar or equivalent to those described herein can be used in the practice of the present invention. Throughout the description and claims the word "comprise" and its variations such as "comprising" are not intended to exclude other technical features, additives, components, or steps. Additional objects, advantages and features of the invention will become apparent to those skilled in the art upon examination of the description or may be learned by practice of the invention. Furthermore, the present invention covers all possible combinations of particular and preferred embodiments described herein. The following examples and drawings are provided herein for illustrative purposes, and without intending to be limiting to the present invention.

### EXAMPLES

### EXAMPLE 1: Production of mAb, independent HC and independent LC

Anti-HER2 mAb, anti-HER2 LC and HC were independently produced in HEK293 cultures and purified by affinity chromatography.

### HEK293 cultures and mAb, HC and LC expression

Trastuzumab protein sequence for LC (SEQ ID NO: 1) and HC (SEQ ID NO: 2) were obtained from the Drugbank website (ref. DB00072). The optimized signal peptides for trastuzumab were added to LC (SEQ ID NO: 3) and HC (SEQ ID NO: 4) (Haryadi R et al., 2015). The Trastuzumab DNA sequence for both LC (SEQ ID NO: 5) and HC (SEQ ID NO: 6) were obtained from the mentioned protein sequence, after being optimized to mammalian codon usage using the Codon Optimization On-line (COOL) method (Chin J et al., 2014), and were synthesized by Genscript (NJ, USA).

HEK293SF-3F6 producing Anti-HER2 were obtained by cloning the above synthetic DNA sequence in a tricistronic expression vector derived from the commercial pIRESpuro3 vector (Clontech, CA, USA) between Nhel-Agel for light chain DNA sequence and BamHI-EcoRI for heavy chain DNA sequence. HEK293SF-F6 cells were then transfected with the construct and selected by puromycin antibiotic resistance. The new anti-HER2 antibody producer cell lines were referred to as HEK293_T10.

HEK293SF-3F6 producing anti-HER2 HC or anti-HER2 LC were obtained by cloning the above-mentioned synthetic DNA sequences in the expression vector pIRESpuro3 (Clontech, CA, USA) between BamHI and Notl for HC and in the vector pIRESneo3 (Clontech) between Nhel and Agel for LC. HEK293 cells were then transfected with the different constructs and selected by antibiotic resistance; puromycin was used for HC and neomycin for LC. The new producer cell lines were referred to as HEK293_THC and HEK293_TLC.

Cells were subcultured at 0.3×10⁶ cell/mL three times per week to keep them in exponential growing phase. Cell maintenance was performed in 125 mL polycarbonate shake flasks (Corning Inc., NY, USA), with a working volume of 12 mL, and maintained at 37°C in an incubator with a 5% CO₂ humidity saturated atmosphere (Steri-cult 2000 Incubator, Forma Scientific). Flasks were continuously agitated at 110 rpm on an orbital shaking platform (Stuart SSL110).

Culture media SFM4Transfx-293 (HyClone, UT, USA) supplemented with 4 mM GlutaMAX (Gibco, Invitrogen, CA, USA), 10% (v/v) Cell Boost 5 (60 g/L solution) (HyClone), 2% (v/v) Kolliphor P188 (100 g/L solution) (Sigma, MO, USA) and 0.5% (v/v) Antifoam C (10 g/L solution) (Sigma) was used in shake flasks and 5 L wave bag cultures for HEK293_T10, HEK293_THC and HEK293-TLC cell lines. Selection pressure was maintained during the production step by adding 0.2% (v/v) puromycin (1 mg/mL) (Merck, NJ, USA) for HEK293_T10 and HEK293_THC and 20 mL/L of neomycin (G-418 solution, Roche, Basel, Switzerland) for HEK293_TLC.

Bioreactor cell cultures were performed using Flexsafe RM 10L bags (DFB010L, Sartorius, Göttingen, Germany) in a WAVE 20/50 EHT (GE Healthcare, IL, USA) with a working volume of 5 L. Temperature was set at 37°C. Agitation and angle were set at 22 rpm and 8°C, respectively.

### HC and LC purification by affinity chromatography

Purification steps included solids separation by depth filtration (Clarisolve 40MS, Merck) and a bioburden reduction filter (Millipore Express SHF, Merck), broth concentration by tangential flow filtration (Hydrosart 30 KDa membrane, Sartorius), protein A affinity chromatography for mAb and HC and purification (MAb Select Sure, GE) and protein L affinity chromatography for LC purification (Capto L, GE), as described by the supplier and buffer exchange by desalting columns (PD Minitrap G-25, GE) for mAb, HC and LC. The final buffer was 50 mM sodium citrate pH 6 for mAb, HC and LC.

### Analytical methods

SDS-PAGE gels: Protein mixture composition was analysed by SDS-PAGE gels (MiniProtean TGX Stain-free gels 4-20%, Biorad, CA, USA). Bands densitometry to determine the relative amount of each band in the sample were analyzed with the software GelDoc EZ (Biorad).

SEC-HPLC: SEC experiments were performed in Waters Alliance 2695 system using Zenix-C SEC-300 (4.6 x 300 mm, Sepax, DE, USA). The mobile phase was PBS 1x (Sigma) at a flow rate of 0.35 ml/min. The UV absorbance was measured at a wavelength of 214 nm.

### Results and Conclusions

SDS-PAGE analysis shows (FIG. 2) that under denaturing and non-reductive conditions, HC is detected as a single band of 100 KDa aprox., which means that HEK293_THC express the protein forming a dimer. Results were confirmed by SEC-HPLC (FIG. 3A). Unexpectedly, LC appears in 2 bands in the SDS-PAGE gel, representing 55% the 42 KDa band and 45% the 21 KDa band, corresponding to a LC dimer covalently bonded and to LC monomers, respectively. However, SEC-HPLC analysis shows that there is only one peak of 42 KDa under native conditions (FIG. 3B). Therefore, anti-HER2 LC was expressed forming dimers, but whereas 55% of them are covalently bonded, 45% of LC was forming dimers without covalent unions. 3% of aggregates were observed in the produced LC.

### EXAMPLE 2: mAb assembly from independently produced HC and LC. Approach 1 (reduction-oxidation)

Prior to conjugation, the mAb assembly was evaluated following a sequential reduction-oxidation protocol of LC and HC. The mAb assembly approach 1 (reduction-oxidation) shown in FIG. 4 consists of a mixture of HC and LC. Later, reduction of the disulfide bonds of the mixed chains was achieved by the addition of DTT and the mixture was incubated at 37°C for 1 h. Next, a buffer exchange column allowed glutathione/cysteine and DTT removal while the product remained in 50 mM citrate buffer. Finally, dhAA was added to promote disulfide bonds re-oxidation for 3 hours at room temperature. An excess of HC was applied in the mixture, rendering LC the limiting reactant.

### Analytical methods

ELISA assay: Assembly was evaluated by comparing *in vitro* isolated anti-HER2 recognition. Namely, 50 µL of 50 ng/µL anti-HER2 in PBS solution (SinoBiological, Beijing, China) was placed in the wells of 96-well Maxisorp plates for its adsorption at 4°C overnight. After removal of the supernatants, 100 µL of 2% skim milk were added and allowed to stand at room temperature for 1 h. The plates were washed three times with PBS-Triton and 50 µL of samples at different dilutions in PBS were placed in each well. Samples were analyzed in duplicates. After standing at room temperature for 1 h, the plates were washed three times with PBS-Triton. After the addition of 50 µL of a solution of 0.1 µg/mL of polyclonal anti-IgG1 conjugated to Horseradish peroxidase (Genscript), the plates were allowed to stand at room temperature for 1 h. After three washings with PBS-Triton, 50 µL of TMB were added. The staining reaction was carried out at room temperature for 10-15 minutes and stopped by adding 50 µL of 20% sulfuric acid addition. The absorbance at 450 - 630 nm was measured with a Labtech LT-4000 microplate reader (Labtech, MI, USA).

SEC-HPLC: details are described in EXAMPLE 1.

### Results and conclusions

SEC-HPLC results shown in Table 1 demonstrate that a 73% of mAb was successfully reassembled.

| **Table 1. Molecules detected under native conditions (SEC-HPLC)** | | | | |
|---|---|---|---|---|
| Molecule | Aggregates | Monomer mAb | dcHC | dcLC |
| Reduction-oxidation approach (% Area) | 2.1 | 73.0 | 14.5 | 10.4 |
| Spontaneous assembly approach (% Area) | 8.0 | 85.3 | 6.7 | ND |

ELISA test confirmed that the assembled mAb recognized HER2 antigen, while isolated HC or LC showed no significant signal of HER2 antigen recognition (Table 2).

| **Table 2. Isolated antigen HER2 recognition in the ELISA test** | | | | | |
|---|---|---|---|---|---|
| Molecule | *In vivo* folded LC | *In vivo* folded HC | *In vitro* reassembled trastuzumab (red-ox approach) | *In vitro* reassembled trastuzumab (spontaneous approach) | *In vivo* folded trastuzumab (control) |
| Isolated antigen HER2 recognition | - | - | + | + | + |

### EXAMPLE 3: mAb assembly from independently produced HC and LC. Spontaneous approach

The mAb assembly was evaluated following an alternative approach without a reduction-oxidation procedure. Based on the empirical proportion of LC and HC determined in the red-ox assembly approach, a simpler approach was explored consisting in the mixture of LC and HC in 50 mM citrate pH 6 buffer for 3 hours at room temperature.

### Analytical methods

SEC-HPLC: details are described in EXAMPLE 1.

ELISA assay: details are described in EXAMPLE 2.

### Results and conclusions

Isolated HC and LC were mixed in 50 mM citrate pH 6 buffer in the same proportion and similar results were obtained without a reduction-oxidation procedure. In this approach, no disulfide bonds were generated, but mAb original structure was assembled through non-covalent interactions, since SEC-HPLC profile confirmed the presence of 85.3% of monomer (Table 1) and the resulting product recognized isolated HER2 antigen in an ELISA assay in the same proportion as *in-house* anti-HER2 antibody (Table 2) produced by HEK293 by using a tricistronic vector, as described in the EXAMPLE 1.

In the red-ox approach, a lower yield was obtained, since residual LC was detected in the final product. The presence of both residual HC and LC forces a more complex purification sequence than in spontaneous approach, where only residual HC was detected.

The final mixture of monomer, aggregates and dcHC obtained in the spontaneous assembly approach was purified by Capto L affinity chromatography (specific for LC), since no residual LC was detected by SEC-HPLC so only the assembled mAb would be captured.

### EXAMPLE 4: ADC assembly from independently produced HC and MMAE-conjugated LC

The easiest strategy for mAb assembly was the assembly approach 2 (spontaneous approach) and it was applied to MMAE-conjugated LC. Since DTT reduction power is not optimal at pH 6, TCEP was used to reduce purified LC (FIG. 5). Under this condition, LC was not forming covalently bonded dimers (analyzed by SDS-PAGE) and was conjugated to MC-vc-PAB-MMAE construct (MedchemExpress, NJ, USA) (construction MC-vc-PAB-MMAE is referred in this description simply as "MMAE").

Working at a concentration range of 1-5 mg/mL of HC in the final mixture led to a successful reassembly of the mAb. In this work a relation of 5.54 g dcHC/g scLC was applied. Thereafter, protein A affinity chromatography (GE) was used in the ADC purification so unassembled LC-MMAE does not bind to protein A ligand.

### Analytical methods

HIC-HPLC: HIC experiments were performed using a Waters Alliance 2695 system using Proteomix HIC Butyl-NP5 (4.6 x 50 mm, Sepax). The mobile phase was a gradient of 25 mM sodium phosphate pH 7 (Sigma) with decreasing ammonium sulphate (2170, Merck) concentration (1.8 M to 0 M) and increasing concentration of isopropanol (Scharlab, Barcelona, Spain) to improve peaks resolution, as recommended by the column manufacturer, at a flow rate of 0.8 ml/min. The UV absorbance was measured at a wavelength of 214 nm.

SDS-PAGE gels and SEC-HPLC: details described in EXAMPLE 1.

ELISA assay: details are described in EXAMPLE 2.

### Results and conclusions

Purified LC was completely reduced with TCEP and conjugated with MC-vc-PAB-MMAE in the only solvent accessible thiol group of cysteine in the LC. Since there is only one thiol available, each LC was conjugated to one MC-vc-PAB-MMAE molecule and a homogeneous solution of LC-MMAE was obtained, as checked by HIC-HPLC (FIG. 6).

FIG. 6 shows the differences in hydrophobicity between LC and conjugated LC due to the presence of the hydrophobic cytotoxic drug. A single peak was detected in the conjugated LC, showing a conjugation efficiency close to 100%.

The applied assembly strategy for ADC generation was the spontaneous approach for its simplicity and efficacy. The ADC and the correspondent assembly process control mAb were characterized in terms of purity and HC was checked to be the limiting reactant with both SEC-HPLC (FIG. 7) and HIC-HPLC (FIG. 8). ADC was positively more hydrophobic than anti-HER2 (FIG. 8) due to the cytotoxic drug presence in LC.

Before further characterization of the products obtained, both ADC and assembled anti-HER2 were purified by protein A affinity chromatography, which is specific for HC and only the assembled mAb or ADC would be captured. In terms of antigen recognition, the obtained monomer recognized isolated HER2 antigen in an ELISA assay in the same proportion as in-house anti-HER2 antibody produced by HEK293 by using a tricistronic vector, as described before in EXAMPLE 1 and shown in Table 3.

| **Table 3. Isolated antigen HER2 recognition in the ELISA test** | | |
|---|---|---|
| Molecule | Homogeneous T-MMAE DAR 2 | *In vivo* folded trastuzumab (control) |
| Isolated antigen HER2 recognition | + | + |

### EXAMPLE 5: Biological activity of DAR 2 homogeneous T-MMAE

HER2-positive breast cancer cells SKBR3 were used to assess the cytotoxicity of the homogeneous DAR 2 T-MMAE (trastuzumab conjugated to MMAE) previously obtained. Its biological activity was compared to an *in-house* heterogeneous DAR 4 T-MMAE (control) using an *in vitro* MTS cell-based assay.

### Heterogeneous T-MMAE DAR 4 (control for MTS test)

Heterogeneous T-MMAE DAR 4 was obtained by trastuzumab reduction with 4:1 molar proportion of TCEP with respect to trastuzumab followed by the addition of DTPA (diethylenetriaminepentaacetic acid) at a final concentration of 1 mM. Next, the conjugation step occurred by the reaction of free thiol groups of reduced trastuzumab to MC-vc-PAB-MMAE (MedchemExpress), used in a 10:1 molar proportion.

### MTS assay

SK-BR-3 cells at the logarithmic growth phase were washed with sterile PBS 1x (HyClone), treated with trypsin (Sigma), re-suspended in 10% Fetal Bovine Serum (Sigma) DMEM medium (Sigma) with cell density at 2x10⁵ cells / ml and seeded in a 96-well cell culture plate (Corning) by applying 50 µL of cell suspension per well. The plates were incubated at 37°C in a humidified 5% CO₂ incubator for 24 hours. Next, samples to be analyzed were diluted to the concentration range to be tested and 50 µL were applied to the cell culture wells under sterile conditions by using decreasing sample concentrations. An entire cell culture plate was required to analyze 1 sample because 7 replicates were analyzed per concentration. The last row of each plate was used as control, which consisted of cells in medium without sample to be tested. The plates were incubated at 37°C in a humidified 5% CO₂ incubator for 72 hours. After incubation, each well was treated with 20 µL MTS solution (Promega, WI, USA) followed by incubation for 3 hours. The absorbance at 405 nm was measured with a Labtech LT-4000 microplate reader.

### Results and conclusions

The results obtained (FIG. 9) show that homogeneous DAR 2 T-MMAE had an antiproliferative effect on the target cells and it exhibited a significantly lower cytotoxic activity than DAR 4 heterogeneous T-MMAE. These differences correspond to the differences in the drug load. Furthermore, *in vitro* assembled mAb behaved similarly to trastuzumab folded *in vivo* (FIG. 9).

### EXAMPLE 6: In vivo assay

The efficacy of the ADC from EXAMPLE 4 in tumor growth inhibition was tested in an orthotopic model of human BT747 breast cancer cells in athymic nude mice.

### Tumor growth inhibition

BT474 human breast cancer cells were inoculated i.m.f.p into the right abdominal mammary fat pad in athymic nude mice. Once tumors were established, intravenous treatment with the ADC or the vehicle was started in a 2-dose administration. Thereafter, tumor volume, body weight and the appearance of clinical adverse effects were monitored along time. At the end time point, mammary primary tumor and main organs (liver, kidneys, spleen, lungs and heart) were excised and collected.

### Results

Tumors were monitored twice or three times a week over time. At end-point (day 23), only 33% of the animals in the ADC group showed measurable tumors, while 100% animals in the vehicle group had measurable tumors. These results indicate that the treatment was extremely effective (FIG. 10). Such efficacy was accompanied with significant although manageable weight loses as secondary side-effect in test compound-treated animals.

### EXAMPLE 7: mAb and ADC analysis

Mass spectrometry of mAb, ADC was performed in order to analyze the nature of the molecules involved in the study.

### MS analysis

mAb and ADC were analyzed by manual desalting/native MS using SynaptG2 (Q-TOF). Desalting was performed in 30 KDa Vivaspin columns (Sigma). Original buffer was substituted by ammonium acetate (pH 6.8). Then the sample was analyzed by Native MS using Synapt G2 Q-TOF (Waters) at 180 V and 6 mbar. Data processing was carried out using MassLynx software.

### Results

Results obtained confirm that only DAR 2 is detected in the ADC. Average DAR of site-specific ADC is 2.0 and conjugation is located to LC. Other glycosylated 100 KDa species were also detected in mAb and in ADC samples (FIG. 11).

### REFERENCES

### Non-patent literature:

Jackson DY. "Processes for constructing homogeneous antibody drug conjugates" Org. Process Res. Dev 2016, 20, 5, 852-866.
Haryadi R et al., "Optimization of heavy chain and light chain signal peptides for high level expression of therapeutic antibodies in CHO cells", PLoS One 2015, 10(2): e0116878.
Chin J et al., "Codon optimization online (COOL): a web-based multi-objective optimization platform for synthetic gene design", Bioinformatics 2014, 30(15), 2210-2212.

## Claims

1. A method for producing antibody conjugates having homogeneous payload antibody ratio, the method comprising:
i) providing separate fractions of heavy chains and light chains of an antibody or a fragment thereof both having interchain thiols;
ii) separately conjugating a payload to the interchain thiols of the heavy chains or to the interchain thiols of the light chains or to both; and
iii) assembling the resulting heavy chains with the resulting light chains by mixing them to form antibody conjugates.

2. The method for producing antibody conjugates according to claim 1, wherein the step (i) of providing separate fractions of heavy chains and light chains is performed by a method selected from:
a) separately obtaining heavy chains and light chains; and
b) obtaining a complete antibody, separating the heavy chains and the light chains under reduction and denaturalization conditions, and separately refolding the heavy chains and the light chains.

3. The method for producing antibody conjugates according to claim 1, wherein the resulting antibody conjugates are homogeneous with a payload antibody ratio of 2 and wherein a payload is conjugated to the interchain thiols of the light chains, the method comprising:
i) providing separate fractions of heavy chains and light chains;
ii) providing light chains with free interchain thiols;
iii) conjugating the payload to the free interchain thiols of the light chains; and
iv) assembling the conjugated light chains obtained in step (iii) with the fraction of unconjugated heavy chains by mixing, to form antibody conjugates.

4. The method for producing antibody conjugates according to claim 1, wherein the resulting antibody conjugates are homogeneous with a payload antibody ratio of 2 and wherein a payload is conjugated to the Fab domain interchain thiols of the heavy chains, the method comprising:
i) providing separate fractions of heavy chains and light chains;
ii) providing heavy chain dimers with interchain disulfide bonds between the monomers, and with the Fab domain interchain thiols in free form;
iii) conjugating the payload to the Fab domain interchain thiols in free form of the heavy chain dimers; and
iv) assembling the conjugated heavy chain dimers obtained in step (iii) with the fraction of unconjugated light chains by mixing, to form antibody conjugates.

5. The method for producing antibody conjugates according to claim 1, wherein the resulting antibody conjugates are homogeneous with a payload antibody ratio of 4 and wherein a payload is conjugated to the Fab domain interchain thiols of the heavy chains and a payload is conjugated to the interchain thiols of the light chains, the method comprising:
i) providing separate fractions of heavy chains and light chains;
ii) providing light chains with free interchain thiols;
iii) conjugating a payload to the free interchain thiols of the light chains;
iv) providing heavy chain dimers with interchain disulfide bonds between the monomers, and with the Fab domain interchain thiols in free form;
v) conjugating a payload to the Fab domain interchain thiols in free form of the heavy chain dimers; and
vi) assembling the conjugated heavy chain dimers obtained in step (v) with the conjugated light chains obtained in (iii) by mixing, to form antibody conjugates;
wherein the payload conjugated to the light chains is the same or different from the payload conjugated to the heavy chains.

6. The method for producing antibody conjugates according to claim 1, wherein the resulting antibody conjugates are homogeneous with a payload antibody ratio of 8 and wherein a payload is conjugated to interchain thiols of the heavy chains and a payload is conjugated to the interchain thiols of the light chains, the method comprising:
i) providing separate fractions of heavy chains and light chains;
ii) separately providing light chains and heavy chains with all interchain thiols in free form;
iii) conjugating a payload to the interchain thiols in free form of the heavy chains;
iv) conjugating a payload to the free interchain thiols of the light chains; and
v) assembling the conjugated heavy chains obtained in step (iii) with the conjugated light chains obtained in (iv) by mixing, to form antibody conjugates;
wherein the payload conjugated to the light chains is the same or different from the payload conjugated to the heavy chains.

7. The method for producing antibody conjugates according to any of claims 3, 5 and 6, wherein the step of providing light chains and/or heavy chains with free interchain thiols, comprises the steps of:
a) reducing the fraction of light chains or heavy chains with a reducing agent; and
b) in case of excess of reducing agent, eliminating the reducing agent.

8. The method for producing antibody conjugates according to any of claims 4-5, wherein the step of providing heavy chain dimers with interchain disulfide bonds between the monomers, and with the Fab domain interchain thiols in free form, comprises the steps of:
a) reducing the fraction of heavy chains with a reducing agent;
b) in case of excess of reducing agent, eliminating the reducing agent; and
c) reoxidizing the resulting fraction of heavy chains.

9. The method for producing antibody conjugates according to any of claims 1-8, wherein the payload is selected from the group consisting of a drug, an oligonucleotide, a peptide, an enzyme, a protein, a substrate, an inorganic compound, a chelate, a radioactive agent, a detectable reagent, and a nanoparticle.

10. The method for producing antibody conjugates according to any of claims 1-9, wherein the separate fractions of heavy chains and light chains are obtained by a method comprising:
a) separately growing expression cells which comprise a DNA construct with the heavy chain sequence or the light chain sequence;
b) separately expressing the heavy chains and the light chains; and
c) separately purifying the heavy chains and the light chains, comprising solids separation and chromatography, to obtain separate fractions of heavy chains and light chains.

11. A homogeneous composition obtainable by any of the methods as defined in claims 1-10, comprising antibody conjugates of the same payload antibody ratio, wherein the antibody in the antibody conjugate has its native amino acid sequence without modifications.

12. The homogeneous composition according to claim 11, wherein the antibody conjugates have a payload antibody ratio of 2 and the payload is conjugated to the Fab domain interchain thiols of the heavy chains or of the light chains.

13. The homogeneous composition according to claim 11, wherein the antibody conjugates have a payload antibody ratio of 4 and the payload is conjugated to both the Fab domain interchain thiols of the heavy chains and the light chains.

14. The homogeneous antibody conjugates according to claim 13, wherein the payload conjugated to the light chains is different from the payload conjugated to the heavy chains.

15. The homogeneous composition according to any of claims 12-13, wherein the antibody is trastuzumab and the payload is monomethyl auristatin E linked via a valine-citrulline dipeptide.
